(19) Europäisches Patentamt
European Patent Office
Office européen des brevets

(11) Numéro de publication: 0 329 501
A1

(12) **DEMANDE DE BREVET EUROPEEN**

(21) Numéro de dépôt: 89400218.7

(22) Date de dépôt: 26.01.89

(51) Int. Cl.[4]: C 12 N 15/00
C 12 N 1/16, C 12 P 21/02

(30) Priorité: 28.01.88 FR 8800973

(43) Date de publication de la demande:
23.08.89 Bulletin 89/34

(84) Etats contractants désignés:
AT BE CH DE ES FR GB GR IT LI LU NL SE

(71) Demandeur: INSTITUT NATIONAL DE LA RECHERCHE AGRONOMIQUE (INRA)
145, rue de l'Université
F-75341 Paris Cédéx 07 (FR)

(72) Inventeur: Fournier, Philippe
23, avenue René Coty
F-75014 Paris (FR)

Gaillardin, Claude
7, Allée des Gardes Royales
F-78000 Versailles (FR)

Kudla, Bernard
149, rue Raymond Losserand
F-75014 Paris (FR)

Heslot, Henri
29, rue Rousselet
F-75007 Paris (FR)

(74) Mandataire: Warcoin, Jacques et al
Cabinet Régimbeau 26, avenue Kléber
F-75116 Paris (FR)

Les microorganismes ont été déposés auprès de la Collection de Culture de Microorganismes de l'Institut Pasteur sous les numéros I-724, I-725, I-726.

(54) **Séquence ars efficace chez yarrowia lipolytica et procédé pour sa préparation.**

(57) La présente invention concerne des séquences ars efficaces dans Yarrowia lipolytica, ainsi que des plasmides portant ces séquences.

Les souches de Y. lipolytica transformées par lesdits plasmides peuvent être utilisées dans des procédés de fermentation permettant de préparer des protéines d'intérêt industriel.

EP 0 329 501 A1

## Description

# SEQUENCE ARS EFFICACE CHEZ YARROWIA LIPOLYTICA ET PROCEDE POUR SA PREPARATION

La présente invention concerne des séquences de réplication autonome chez Yarrowia lipolytica, des procédés pour leur préparation et leur utilisation dans des procédés mettant en oeuvre les ADN recombinants.

L'existence, le rôle et le clonage de séquences conférant l'autonomie de réplication à des plasmides de levure a été très documentée chez Saccharomyces cerevisiae (1,2). Un plasmide bactérien (type pBR322) dans lequel a été cloné un gène marqueur de levure (par exemple un gène codant pour une enzyme biosynthétique, comme LEU2, URA3, LYS2, TRP1, etc.) est capable de s'intégrer à faible fréquence dans le chromosome, le plus souvent par recombinaison homologue au locus considéré. Si on introduit dans un tel plasmide une séquence dite ars (pour autonomously replicating sequence), la transformation revêt alors les caractères suivants :

- haute efficacité (plus de 10 000 transformants/µg d'ADN)
- réplication extrachromosomique des plasmides,
- relative instabilité en mitose.

Ces séquences ars sont des séquences d'ADN soit de la même espèce, soit d'espèces différentes. Elles peuvent être d'origine chromosomique ou extrachromosomique (plasmides, mitochondries ...). Mais les revues réalisées par plusieurs auteurs (1,2) montrent bien que dans la plupart des cas les séquences manifestant une propriété ars dans un hôte hétérologue n'ont pas cette caractéristique dans l'organisme de départ. Dans un contexte homologue, l'hypothèse la plus couramment admise est que les ars isolées du chromosome correspondent aux origines de réplication, qu'on estime espacées en moyenne de 20 à 40 kb sur les chromosomes de levures.

Chez la levure industrielle Yarrowia lipolytica, la transformation intégrative a été décrite avec le gène LYS2 de S. cerevisiae (3) et le gène LEU2 de Y. lipolytica (4). Le promoteur de ce même gène a été utilisé (5) pour diriger l'expression de gènes hétérologues (résistance à la phléomyoine et béta-galactosidase) intégrés dans le chromosome au locus leu2. Par ailleurs, il a été montré (4) que lorsqu'on linéarise le plasmide transformant avec une enzyme de restriction en présentant qu'un site de coupure, on accroît la fréquence de transformation d'un facteur 100 (on passe alors de moins de 100 à plus de 10 000 transformants par µg d'ADN). Cet effet recombinogène des extrémités cohésives générées par enzyme de restriction, déjà connu chez S. cerevisiae, est bien plus notable chez Y. lipolytica.

Quand on veut cloner un gène par complémentation en utilisant la transformation intégrative chez Y. lipolytica, on se heurte aux difficultés suivantes :

(a) la banque d'ADN étant réalisée dans un plasmide intégratif de type pBR322+LEU2, on ignore si les sites de restriction présents une seule fois dans ce plasmide ne sont pas également présents dans les fragments clonés ; par conséquent, pour linéariser l'ADN on est obligé d'opérer des restrictions partielles (et de faire cet essai avec différentes enzymes) dont l'efficacité en transformation est plus faible ;

(b) une fois les clones obtenus par complémentation, il faut procéder à une analyse fine de la carte de restriction du fragment cloné et intégré dans le génome, à l'aide de la technique de Southern et de sondes radioactives ; l'étude de cette carte permet de savoir par quelle enzyme on peut couper l'ADN total du transformant pour ressortir le plasmide, qu'on sélectionne, après ligation, par transformation dans Escherichia coli.

Ces deux inconvénients sont supprimés si on peut réaliser la banque d'ADN dans un plasmide réplicatif contenant une séquence ars ; en effet, il est dès lors inutile de couper le plasmide par enzyme de restriction et celui-ci se maintenant de façon extrachromosomique, peut être aisément récupéré chez E. coli. La présence d'une séquence ars permet en principe également d'amplifier un gène, car les plasmides sont généralement multicopies. Toutes ces raisons ont justifié la recherche d'une ars pour Yarrowia lipolytica.

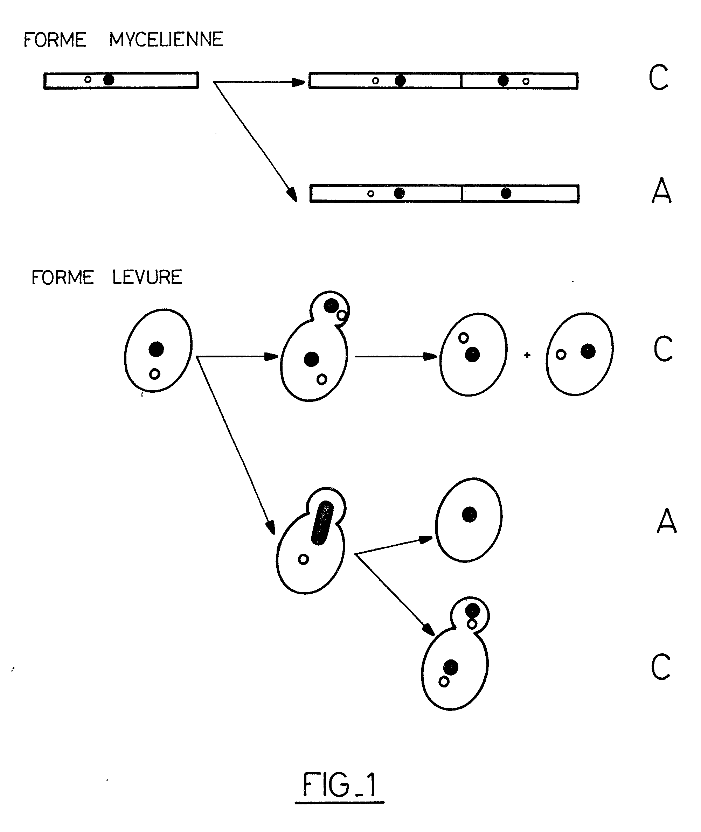

Plusieurs tentatives de sélection s'étant soldées, dans plusieurs laboratoires, par des échecs (6), on a émis l'hypothèse que le mode de division cellulaire de cette levure pourrait en être la cause. En effet, (figure 1) Y. lipolytica est dimorphique, produisant à la fois du mycélium et des formes levuriformes. Il a été montré chez S. cerevisiae (7) que les plasmides ars présentent un fort biais maternel de ségrégation, c'est-à-dire que l'ensemble des copies ont tendance à ségréger comme un bloc et ne passent pas systématiquement à la cellule fille. Si celle-ci est un bourgeon d'une cellule levuriforme, elle va certes cesser de se diviser en milieu sélectif, mais la cellule mère aura encore la possibilité de transmettre le plasmide lors d'une prochaine division (chez S. cerevisiae une cellule mère peut se diviser une vingtaine de fois). En revanche, s'il s'agit d'un article de mycelium, chaque cellule ne donne naissance qu'à une cellule fille. Si le plasmide n'est pas passé lors d'une division, la colonie cesse de se diviser en milieu sélectif. En s'appuyant sur cette hypothèse, on a donc sélectionné des mutants incapables de former du mycelium et on les utilisés pour la recherche de séquences ars. De telles séquences ont été effectivement isolées et il s'est avéré qu'elles étaient aussi fonctionnelles dans des cellules de type mycélien.

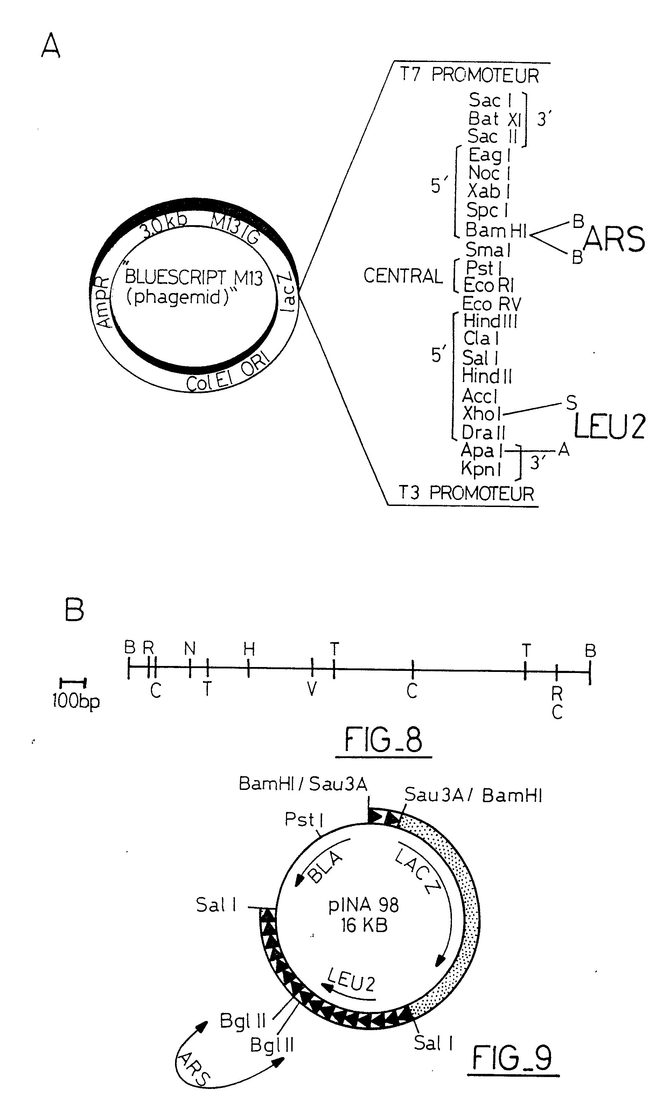

La présente invention concerne une séquence ars efficace dans Yarrowia lipolytica ainsi qu'une séquence d'ADN comportant une telle séquence ars. En particulier, la présente invention concerne la séquence ars efficace dans Y. lipolytica portée par le fragment ADN de 2,2 kb BamHI/BamHI présentant la carte de restriction de la figure 8B, ainsi que les plasmides comportant ces séquences.

La présente invention concerne plus particulièrement un plasmide portant une séquence ars efficace chez Yarrowia lipolytica et qui peut être obtenu notamment par le procédé suivant :

a) on constitue une banque de fragments génomiques de Y. lipolytica dans un vecteur intégratif complémentant une auxotrophie de Y. lipolytica ;

b) on transforme une souche hôte de Y. lipolytica, présentant une auxotrophie pouvant être complémentée par le vecteur intégratif précédent, par les plasmides de la banque précédente ;

c) on hybride sur colonie avec une sonde détectant le vecteur intégratif et on sélectionne les transformants présentant le signal le plus fort ;

d) on effectue un minilysat des clones vecteurs et on met en évidence les plasmides extrachromosomiques ;

e) ces plasmides portent une séquence ars que l'on peut confirmer en testant leur pouvoir de transformation qui est au moins 5 fois supérieur au pouvoir de transformation du vecteur d'origine.

Comme cela sera rappelé dans les exemples, les colonies de Y. lipolytica présentent des morphologies différentes sur le plan macroscopique, à savoir des colonies dites "frisées" ou rugueuses formant des peaux et entourées d'un chevelu à la surface de l'agar, cette morphologie étant plus nette quand les cellules ne sont pas trop nombreuses par boîte, et, d'autre part, des colonies dites "non frisées" qui ne présentent pas ces caractéristiques.

Le phénotype frisé sera noté dans ce qui suit "Fil+" et le génotype correspondant "fil+".

Comme cela ressortira des exemples, dans le cas où la souche réceptrice est une souche Fil-, il est possible entre l'étape b et c de tester la stabilité des transformants et de sélectionner les transformants présentant la plus faible stabilité. En effet, il s'agit du procédé qui a été utilisé pour la sélection des premières séquences ars. Toutefois, lorsqu'on utilise des souches Fil+, il est préférable d'utiliser le procédé décrit précédemment.

Il convient de remarquer, bien entendu, que ce processus peut subir de nombreuses modifications tout en conduisant, dans chaque cas, à la sélection de plasmides portant la séquence ars.

De façon préférentielle, le procédé selon la présente invention sera mis en oeuvre de la façon suivante :

- constitution d'une banque de fragments génomiques d' Y. lipolytica dans un vecteur intégratif, type pINA62 ;
- transformation de plasmides circulaires surenroulés de cette banque dans une souche Fil+, sélection des transformants LEU+ (peuvent contenir un plasmide intégré ou des plasmides réplicatifs) ;
- hybridation sur colonies avec une sonde pBR322 : on s'attend à trouver un signal plus fort dans les transformants réplicatifs multicopie ; isolement de clones ars potentiels ;
- minilysats des clones retenus, transfert de Southern d'ADN non restreint : mise en évidence de plasmides extrachromosomiques avec une sonde pBR322 ;
- transformation d'E. coli avec l'ADN des minilysats et amplification des plasmides ars ;
- caractérisation éventuelle de l'instabilité des clones.

Le processus décrit précédemment correspond, bien entendu, à l'utilisation de vecteurs spécifiques dérivés de pBR322 tels que pINA62.

Les différentes techniques qui sont évoquées dans ce processus, comme dans le processus général, sont bien connues de l'homme de métier et elles seront décrites plus en détail dans les exemples ci-annexés sans que ces caractéristiques de détail puissent être considérées comme déterminantes pour la réalisation de la présente invention.

Comme cela sera également décrit dans les exemples, il est possible, à partir des plasmides comportant les séquences ars, d'effectuer un isolement des séquences ou du moins de séquences plus larges portant la séquence ars. Ces séquences ainsi isolées ou même des fragments des plasmides isolés sont utilisables pour la réalisation de plasmides vecteurs d'expression car la présence de ces séquences ars permet auxdits plasmides de se maintenir à l'état extrachromosomique et de pouvoir être amplifiés, ceci conduisant à une surproduction de la protéine exprimée par ledit plasmide.

Divers vecteurs d'expression ont déjà été décrits dans la technique antérieure, en particulier dans le brevet européen n° 0 220 864 ainsi que les brevets européens n° 0 138 508 et n° 0 166 659.

Ces différents brevets décrivent, pour l'essentiel, des vecteurs dits d'intégration dont on a mentionné préalablement les inconvénients. Toutefois, ces vecteurs d'intégration peuvent être transformés en plasmides extrachromosomiques si on leur adjoint les séquences ars décrites précédemment.

La technologie des vecteurs d'expression ne sera pas décrite en détail, il s'agit, en effet, d'une technologie qui est déjà largement décrite dans les brevets précédents, tant pour ce qui concerne les éléments d'expression des gènes dans Y. lipolytica, en particulier les promoteurs qui peuvent être des promoteurs de Y. lipolytica, par exemple les promoteurs des gènes XPR2 ou LEU2, que pour ce qui concerne les types de gènes qui peuvent être clonés. Parmi les gènes qui peuvent être clonés, il faut citer par exemple les gènes codant pour la prorennine et l'anaphylatoxine C5A humaine, comme cela est décrit dans le brevet européen n° 0 220 864 mais aussi la protéase alcaline extracellulaire (AEP), l'invertase, l'interféron-alpha$_1$ porcin ou la prochymosine.

A titre d'exemple, dans le cadre de la présente invention il sera uniquement fait mention de l'expression de la béta-galactosidase afin de montrer l'efficacité des séquences ars correspondantes, mais il serait évidemment tout à fait possible d'effectuer le même type de construction en utilisant les plasmides décrits dans le brevet européen n° 0 220 864 et en leur adjoignant la séquence ars.

La présente invention concerne donc également, outre les séquences d'ADN portant une séquence ars efficace dans Y. lipolytica, les plasmides incorporant ladite séquence et, en particulier, les plasmides d'expression d'une protéine industrielle.

Ces plasmides d'expression d'une protéine industrielle dans Y. lipolytica comportant, outre la séquence codant pour ladite protéine, l'ensemble des éléments, notamment une région de contrôle comportant un promoteur, permettant d'assurer l'expression desdites protéines dans Y. lipolytica et comportent, en outre, une séquence ars.

Il peut, en outre, être intéressant de disposer d'un plasmide d'intégration comportant ce type de séquence ars et susceptible de complémenter une auxotrophie de Y. lipolytica.

L'invention concerne également la souche de Y. lipolytica transformée par un plasmide tel que décrit précédemment, cette souche pouvant être Fil+ ou Fil-.

Enfin, la présente invention concerne un procédé de préparation d'une protéine industrielle caractérisé en ce qu'on cultive, sur un milieu assurant sa croissance, une souche de Y. lipolytica selon l'invention.

Les exemples ci-après permettront de mettre en évidence d'autres caractéristiques et avantages de la présente invention.

Les figures ci-annexées sont les suivantes :

**Figure 1** : Schéma de transmission d'un plasmide extrachromosomique présentant un biais de ségrégation maternel, dans des cellules à croissance mycélienne ou dans des cellules levuriformes. La croissance (C) ou l'arrêt de la croissance (A) sont dus à la présence (respectivement l'absence) du plasmide (symbolisé par un cercle vide) qui porte le gène marqueur sélectif, le noyau est représenté par le cercle plein.

**Figure 2** : Morphologie des colonies de la souche parente Fil- (INAG33122) et du mutant Fil- (INAG33129), vues de dessus (A) ou du dessous (B). Grossissement 5 à 10 fois.

**Figure 3** : Morphologie des cellules de la souche Fil+ (A) et du mutant Fil-(B) observées au microscope optique (grossissement 400 fois).

**Figure 4** : Carte du Vecteur intégratif pINA62 montrant la stratégie de construction de la banque de fragments BglII (en noir) au site unique BamHI de ce plasmide. La région hachurée est le gène bla de résistance à l'ampicilline, la région en pointillé est le gène LEU2 de Y. lipolytica et ori représente l'origine bactérienne de réplication du plasmide.

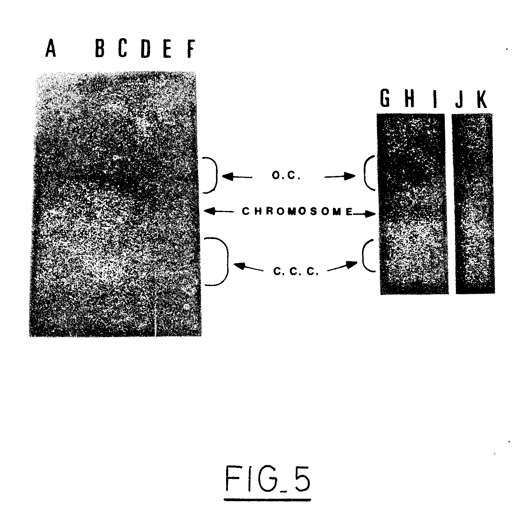

**Figure 5** : Mise en évidence des plasmides dans les transformants instables. L'ADN non restreint de la souche réceptrice Fil- (puits A et K) et de divers transformants (puits B à J) et été hybridé (après migration et transfert sur Biodyne) avec une sonde radioactive (marquée au $^{32}$p) constituée par le plasmide pINA62. Deux ensembles de gels sont présentés ; à gauche on voit les transformants 2-18 (puits B), 2-26 (puits C), 2-39 (puits D), 3-19 (puits E) et 2,75 (puits F) ; à droite, les transformants 2-18 (puits G), 1-68 (puits H), 1-77 (puits I) et 1-27 (puits J). Dans le nom des transformants, le premier chiffre indique le "pool" d'origine et le second est un numéro d'ordre. L'ADN chromosomique est révélé par la partie LEU2 de la sonde sous forme d'une bande diffuse (l'ADN n'étant pas restreint), et est visible dans la plupart des puits (voir en particulier la souche réceptrice où elle constitue le seul signal, puits A et K). Les différences d'intensité reflètent en fait des quantités d'ADN variables déposées sur le gel. Les plasmides sont présents au moins sous forme circulaire ouverte (OC), et plusieurs fois également sous forme CCC. L'observation de la figure permet de distinguer 5 classes de tailles de plasmides, à savoir, dans l'ordre croissant, n° 1 (puits D), n° 2 (puits B, C, F, G), n° 3 (puits H), n° 4 (puits E) et n° 5 (puits I et J).

**Figure 6** : Carte de restriction des 5 plasmides isolés à partir de transformants représentatifs des 5 classes définies à la figure 5, à savoir :

pINA119 provient du transformant 2-18 (classe 2)
pINA123 provient du transformant 1-68 (classe 3)
pINA124 provient du transformant 1-77 (classe 5)
pINA125 provient du transformant 2-39 (classe 1)
pINA126 provient du transformant 3-19 (classe 4)

La partie pBR322 est figurée en noir, le gène LEU2 en pointillé, et l'insertion portant la séquence ars en blanc. Les Plasmides sont représentés ouverts par le site SalI de pBR322, mais la carte SalI n'a pas été établie pour les insertions. les sites de restriction sont les suivants : B = BamHI, G = BglII, E = EcoRI, H = HindIII, B/G = site BamHI détruit par le clonage du fragment BglII. La flèche indique la région homologue (délimitée par deux sites BglII) avec sont orientation.

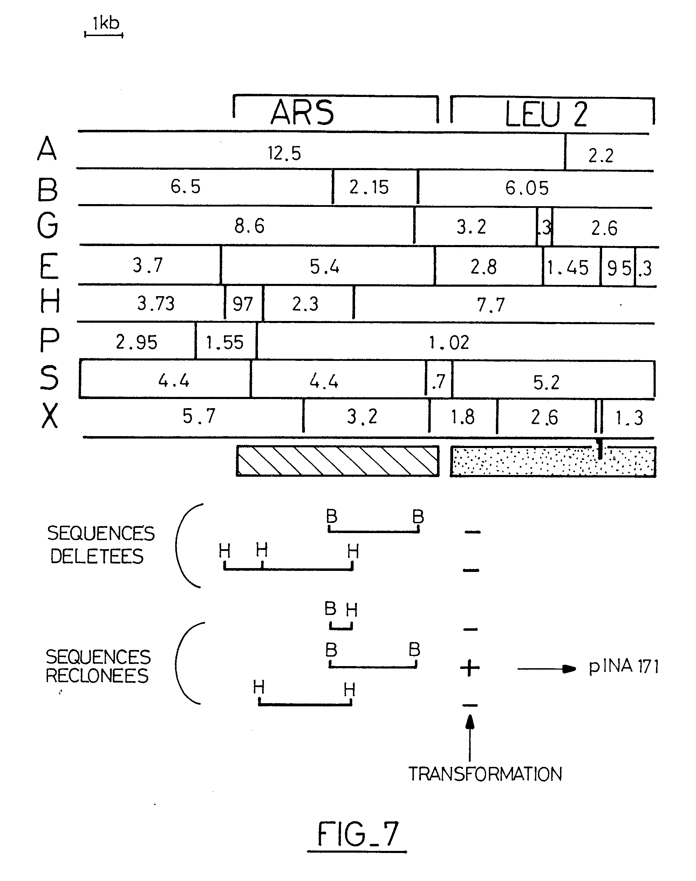

**Figure 7** : Carte de restriction du plasmide ars pINA119. La partie portant la séquence ars est figurée en hachuré et celle portant LEU2 en pointillé. Les dimensions sont à l'échelle (en kilobases). Les enzymes utilisées sont : A = ApaI, B = BamHI, G = BglII, E = EcoRI, H = HindIII, P = PstI, S = SalI et X = XhoI. En bas du diagramme figurent les délétions et les reclonages réalisés et leur efficacité en transformation à haute fréquence chez la souche leu2-35 de Y. lipolytica (avec de l'ADN non restreint). Seul le plasmide pINA171 contenant le fragment BamHI-BamHI de 2,2 kb est efficace.

**Figure 8** : Construction d'un minivecteur pour Y. lipolytica.

A - Ce vecteur de 8,2 kb contient :

. le fragment ars de 2,2 kb (BamHI-BamHI), ce qui permet sa réplication dans Y. lipolytica ;
. le gène LEU2(partie codante sans le terminateur, soit 3 kb de SalI à ApaI) ;
. l'origine de réplication bactérienne colE1, ce qui permet sa propagation dans E. coli ;
. les promoteurs des bactériophages T3 et T7 qui permettent de faire des ARNs à haute efficacité (donc par exemple de synthétiser des sondes radioactives très chaudes) ;
. les séquences de M13 qui facilitent le séquençage (les séquences amorces complémentaires des brins d'ADNs de part et d'autre du multisite sont disponibles commercialement) ;
. un multisite qui permet soit le clonage ultérieur de gènes, soit le démarrage de délétions in vitro, grâce à l'exonucléase III, ou à l'enzyme Bal31 par exemple.
B - Carte fine du fragment ars dans sa région BamHI-BamHI de 2,2 kb (schéma à l'échelle, en paires de bases).
Sur cette figure, les sites de restriction sont symbolisés par : A = ApaI, B = BamHI, C = AccI, H = HindIII, N = NotI, R = RsaI, S = SalI, T = TaqI, V = EcoRV.

**Figure 9** : Expression de la béta-galactosidase. Schéma de construction du plasmide pINA135 à partir du plasmide intégratif pINA98. Le clonage de l'ars entre les deux sites BglII présents sur le fragment SalI-SalI d'ADN de Y. lipolytica qui porte le gène LEU2 est sans effet sur l'expression de ce dernier, car ces sites sont à l'extérieur de la séquence codante proprement dite. On reconnaît le promoteur de LEU2 (fragment Sau3a cloné dans BamHI, voir réf. 5) en amont du gène lacZ.

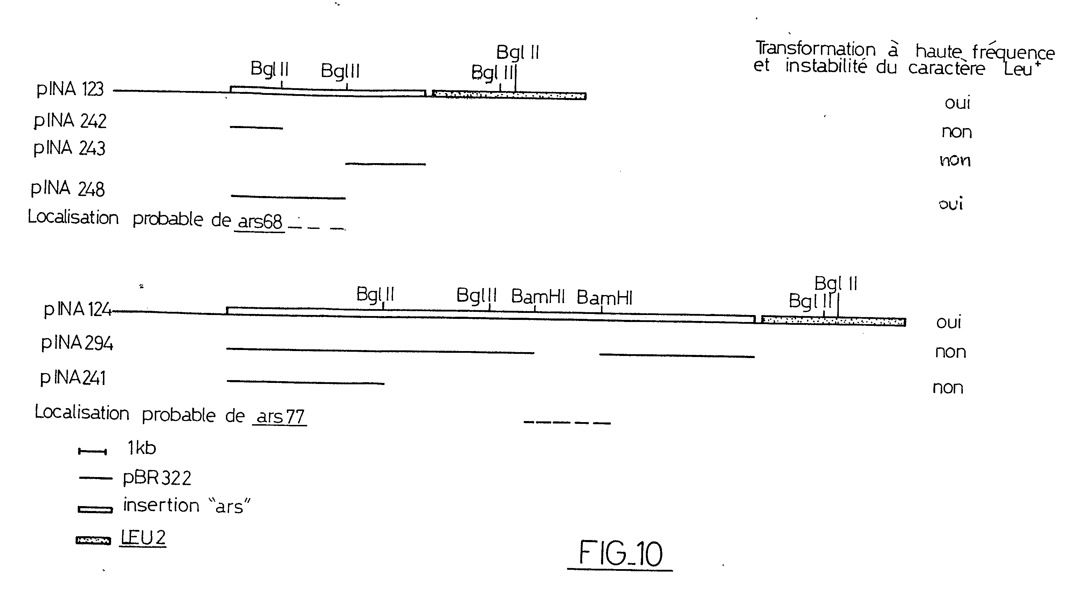

**Figure 10**: Réduction des fragments portant ars68 et ars77. Seules sont représentées les parties qui subsistent dans les plasmides dérivés qui tous contiennent le gène LEU2 comme marqueur de transformation.

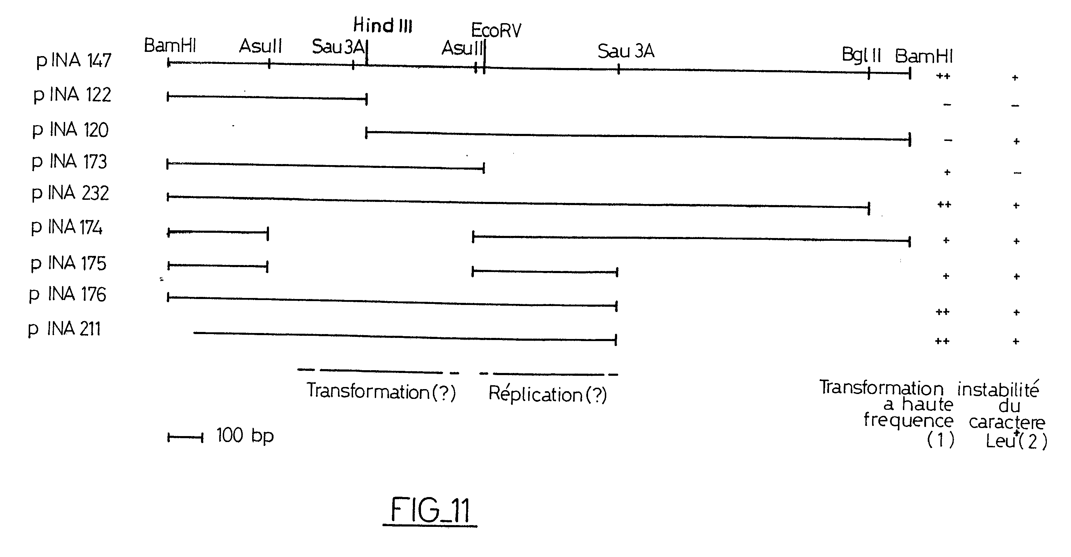

**Figure 11** : Réduction de la séquence ars18. Les plasmides dont les noms figurent à gauche comprennent la partie de l'ars18 soulignée au centre, ainsi que le gène LEU2 de Y. lipolytica, et se trouvent selon les cas dans l'un ou l'autre des vecteurs bactériens pBR322 (pINA122, pINA120, pINA232) ou Bluescript (pINA147, pINA173, pINA174, pINA175, pINA176, pINA211). Tous ont été construits par des délétions ou des reclonages en utilisant les sites de restriction figurés en haute, à l'exception de pINA211 dont la construction est expliquée dans le texte. Les deux régions qui semblent essentielles,.l'une à la transformation à haute fréquence, l'autre à la capacité autoréplicative, sont soulignées au centre.

Notes :

(1) La fréquence de transformation est symbolisée par -- (plus de 5 000 transformants par µg d'ADN), ou par - (200-500 transformants par µg d'ADN), ou par - (moins de 200 transformants par µg d'ADN).
(2) L'instabilité du phénotype $Leu^+$ est notée par + (instable) ou par - (stable).

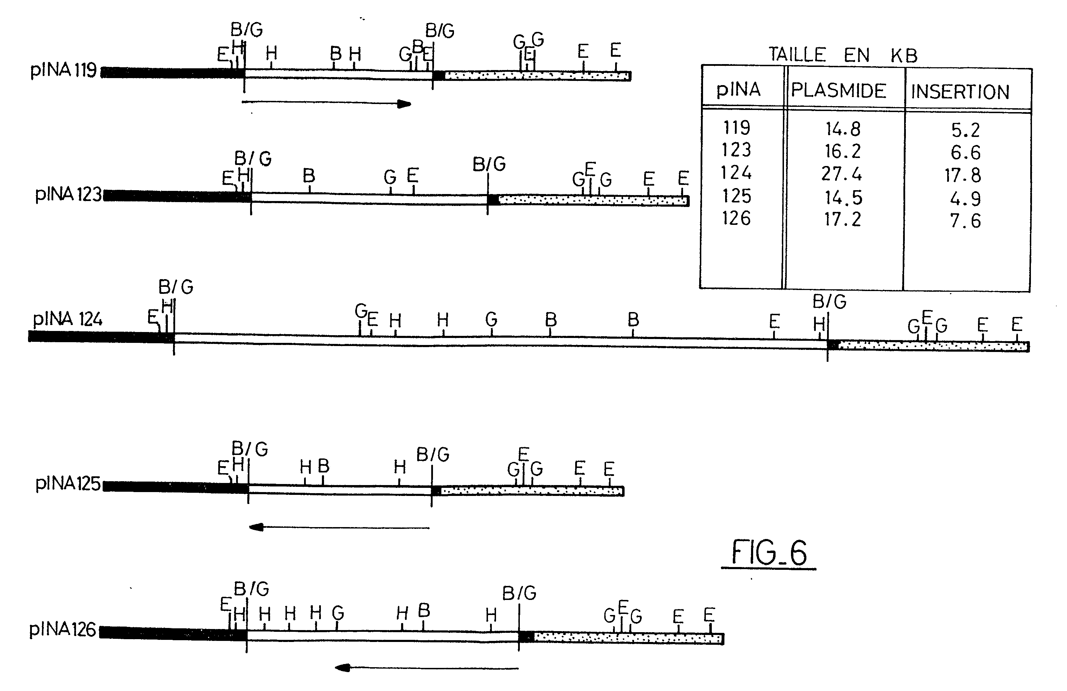

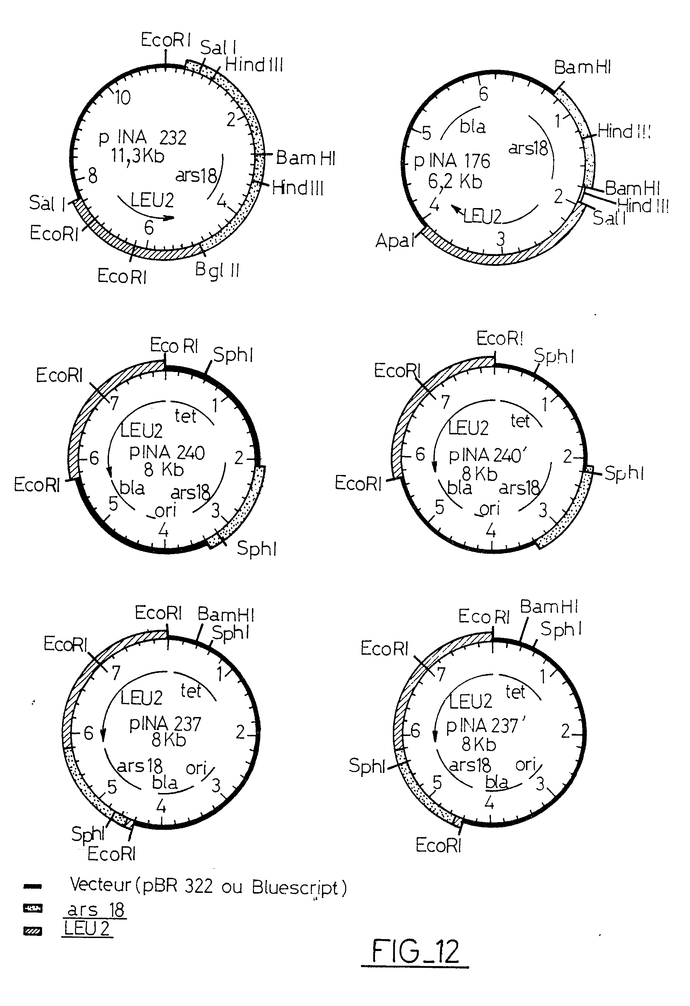

**Figure 12** : Représentation de divers plasmides navettes E. coli-Y. lipolytica portant la séquence ars18. Tous permettent une sélection pour le phénotype $Leu^+$ dans la levure. Le plasmide pINA232 dérive du plasmide pINA119 décrit dans la Figure 6 par une délétion BglII; il porte donc en amont du site unique BamHI une région qui n'est pas nécessaire à la fonction ars. Les cinq autres plasmides possèdent la région ars minimale de 1,3 kb définie à la Figure 11. La partie bactérienne est constituée par Bluescript dans le cas de pINA176, et par pBR322 dans les autres plasmides. Les deux vecteurs pINA237 et pINA237′ dérivent d'un plasmide pBR322 dans lequel on a inséré au site unique EcoRI le fragment de 2298 bp portant le gène LEU2, obtenu par digestion partielle de pINA62; le fragment BamHI de 1,3 kb portant ars18 a ensuite inséré dans les deux orientations entre les deux sites BglII flanquant LEU2. Les plasmides pINA240 et pINA240′ dérivent d'un plasmide pBR322 dans lequel on a inséré au site unique PvuII un lieur BglII (référence Beohringer 909734) qui a permis l'introduction dans les deux orientations du fragment BamHI portant ars18. Le gène LEU2 a été introduit comme ci-dessus dans les plasmides résultants.

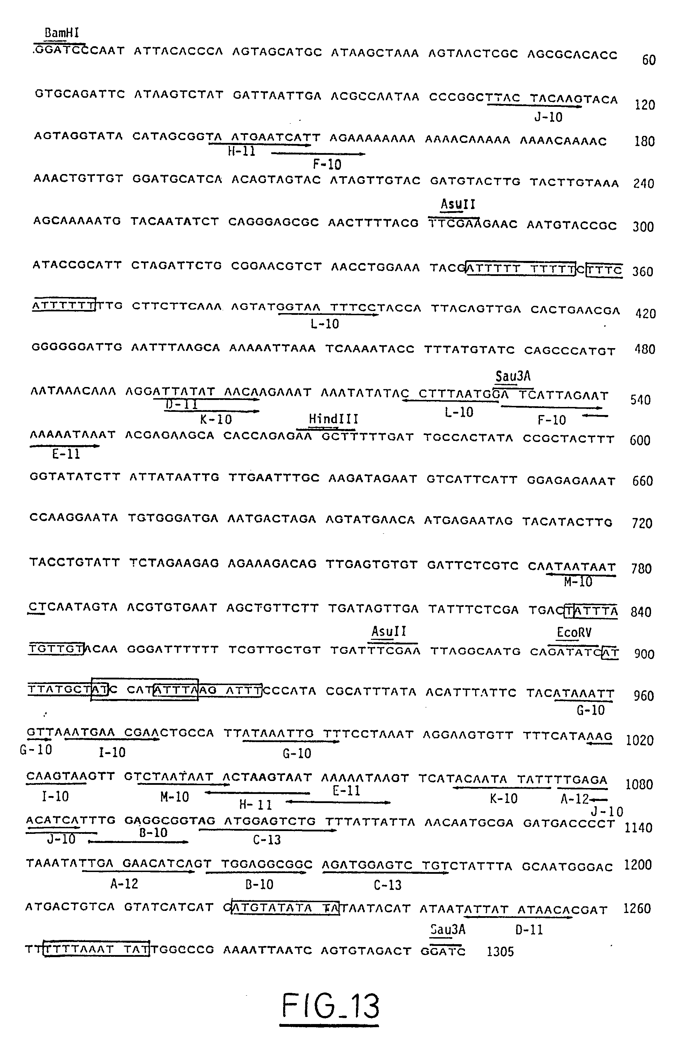

**Figure 13** : Séquence de l'ars18. Les sites de restriction mentionnés à la figure 11 sont reportés au-dessus de la séquence. Les répétitions directes (A à G) et inverses (H à M) comprenant au moins 10 bases sont soulignées et le nombre de bases est indiqué après un tiret. Noter que la répétition K est incluse dans D et que la séquence E est elle-même un palindrome. On remarque également la répétition singulière directe A-B-C (1075-1121 et 1147-1183). Par ailleurs, on trouve des alternances AT (régions 500-520 et 1220-1250), ainsi que des itérations de A (région 150-180) ou de T (région 340-370). Enfin, les séquences encadrées correspondent à des homologies imparfaites avec le consensus d'ars défini chez la levure S. cerevisiae (9 ou 10 bases homologues sur les 11 que comporte le consensus WTTTATPTTTW. où W signifie A ou T et P signifie A ou G, voir réf. 26).

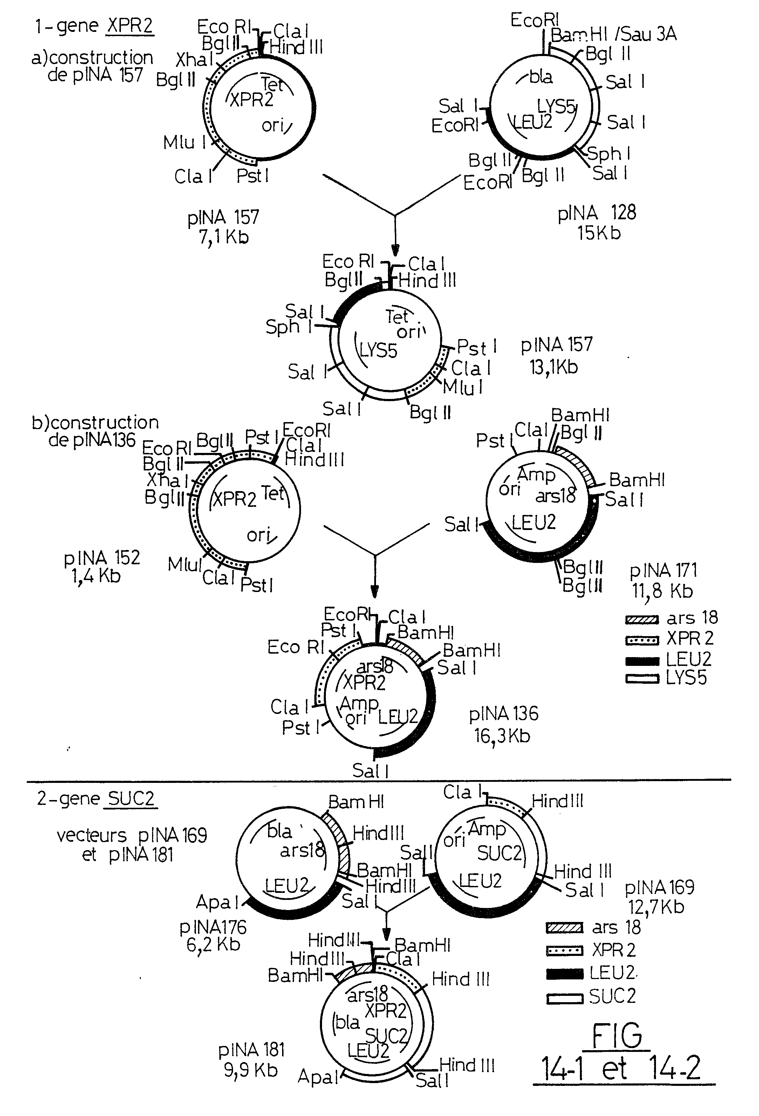

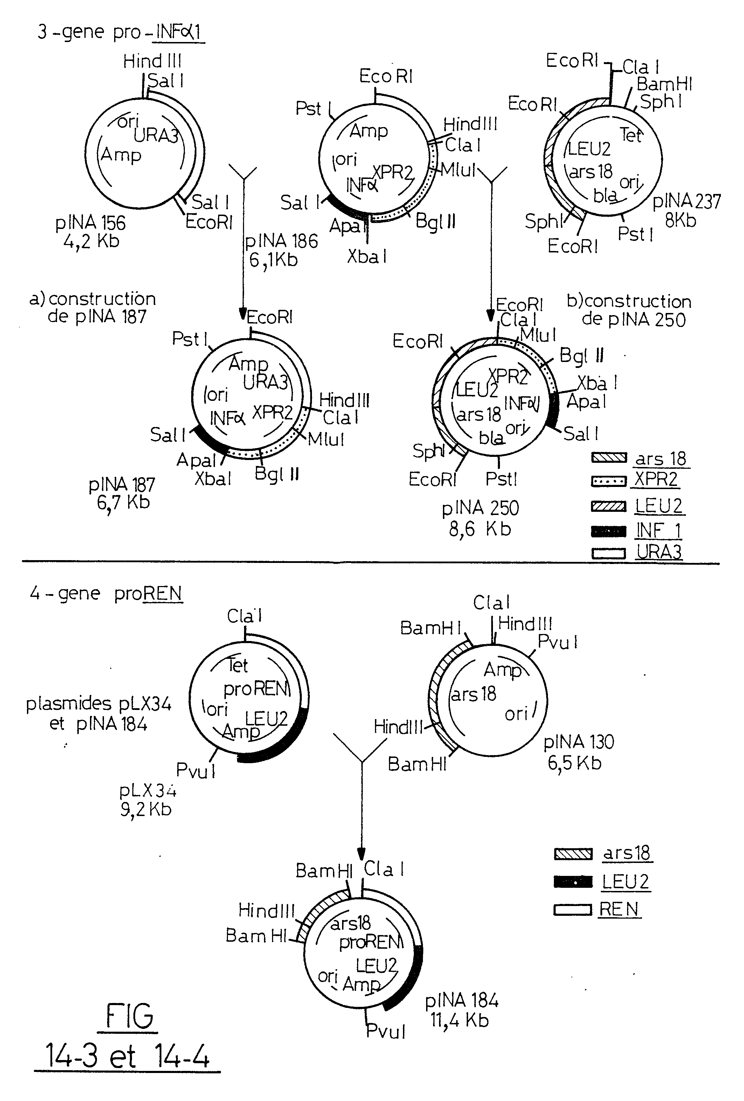

**Figure 14** : Construction des plasmides intégratifs et réplicatifs utilisés pour l'amplification génique.

1) Gène XPR2

a) pINA157 - ce vecteur de disruption a été réalisé par remplacement du fragment BglII (0,9 kb) interne du gène XPR2 par un fragment BglII (6,9 kb) de pINA128 (réf. 14) portant le gène LYS5;
b) pINA136 - ce vecteur réplicatif a été réalisé par insertion d'un fragment ClaI (4,5 kb) de pINA152 contenant le gène XPR2 dans le vecteur réplicatif pINA171 contenant ars18 et le marqueur de sélection LEU2.

2) Gène SUC2

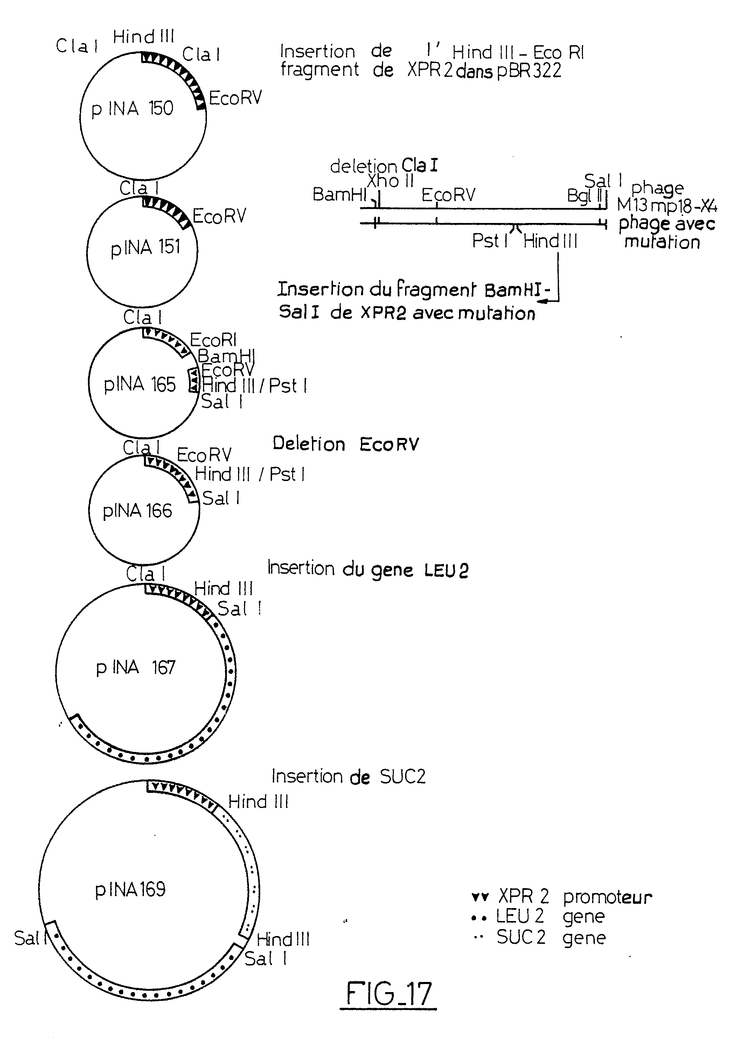

a) pINA169 - vecteur intégratif contenant le gène SUC2 sous le contrôle du promoteur XPR2 (figure 17) et le marquer LEU2. Le vecteur est intégré au site XPR2 dans la souche JM55;
b) pINA181 - vecteur réplicatif contenant le gène SUC2, sous la forme d'un fragment ClaI-SalI de 3,9 kb de pINA169 inséré entre les sites ClaI et SalI du vecteur réplicatif pINA176 (figure 12).

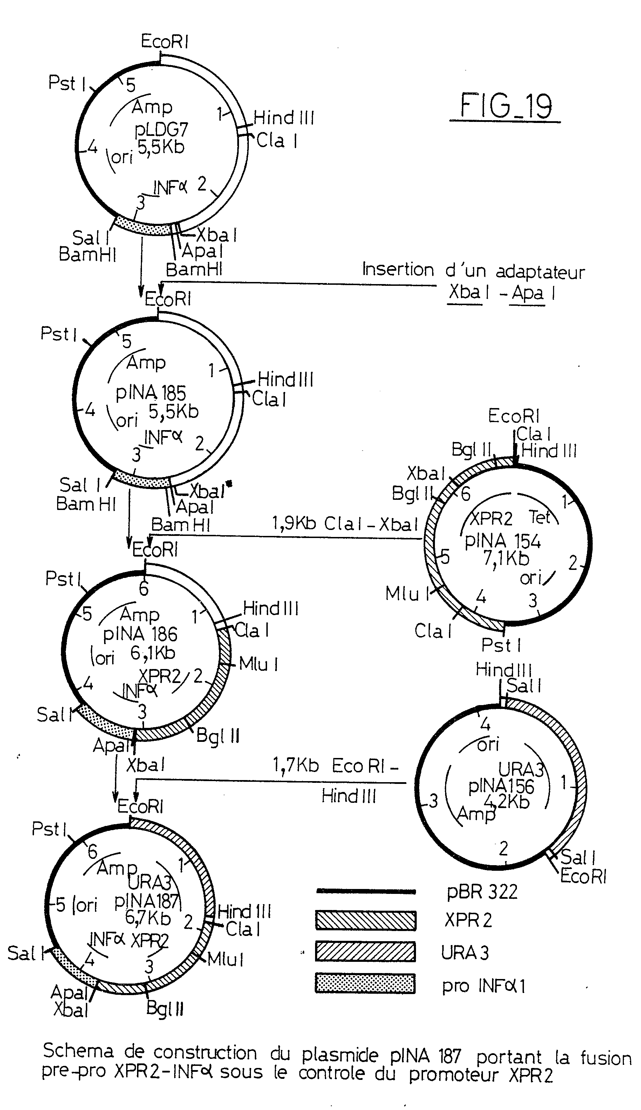

3) Gène pro-INF-alpha$_1$
a) pINA187 - vecteur intégratif contenant le gène pro-INF-alpha-$_1$ de pINA186 (figure 19) et le marqueur URA3 de Y.lipolytica provenant de pINA156. Le vecteur est intégré au site XPR2 dans la souche JM77;
b) pINA250 - vecteur réplicatif contenant le gène pro-INF-alpha$_1$ construit par ligation du fragment ClaI-PstI (4,2 kb) de pINA186 avec le fragment ClaI-PstI (4,4 kb) du vecteur réplicatif pINA237 (figure 12).

4) Gène proREN
a) pLX34 - vecteur intégratif contenant le gène codant pour la prorénine bovine (réf. 20);
b) pINA184 - vecteur réplicatif contenant le gène proREN. Le fragment ClaI-PvuI (5,5 kb) de pLX34 portant à la fois le gène proREN et le marqueur LEU2 a été ligaturé avec le fragment ClaI-PvuI (5,9 kb) de pINA130 contenant ars18, reconstituant ainsi le gène marquer de résistance à l'ampicilline.

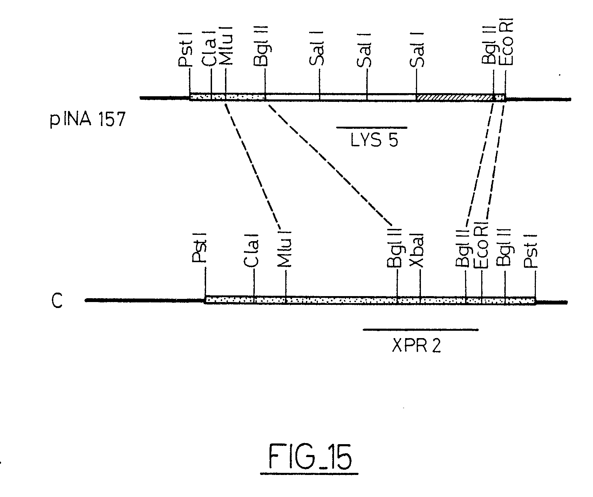

**Figure 15** : Représentation schématique de la construction de la souche JM23. On a représenté la carte de restriction chromosomique du locus XPR2 (C) de la souche JM12 ainsi que la carte de restriction du plasmide de disruption pINA157. Le plasmide de disruption a été préalablement restreint par les enzymes de restriction MluI et EcoRI. On a obtenu par double "crossing over" dans les régions d'homologies MluI-BglII et BglII-EcoRI le remplacement du gène XPR2 par le gène LYS5.

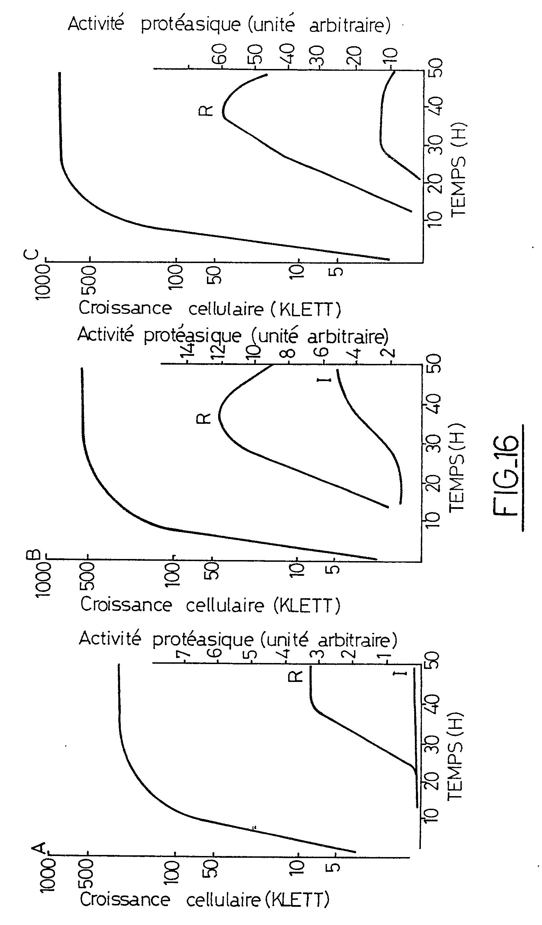

**Figure 16** : Comparaison de la production d'activité protéasique produite au cours de la croissance par la souche JM12 possédant le gène XPR2 intégré (I) et la souche JM70 possédant le gène XPR2 sur le vecteur réplicatif pINA136 (R), cultivées à 28°C en milieux YNB (A), YNBpp (B) et YPDm (C). L'activité protéasique est exprimée en unités arbitraires.

**Figure 17**: Schéma de construction du plasmide pINA169 portant la fusion pre-XPR2-SUC2. La région amont du promoteur XPR2 porté par un fragment HindIII-EcoRV a été inséré dans pBR322 (pINA150). Puis une délétion ClaI permet l'élimination du site HindIII (pINA151). On a, par ailleurs, créé un site HindIII après la région codant pour la séquence signal de la protéase par mutagénèse dirigée. Pour cela le phage M13mp18-X4 portant un fragment XhoI-BglII de 553 bp couvrfant la région autour de l'ATG a été mutagénisé à l'aide de l'oligonucléotide mutagène :
(5'CAGCATCAGAAGCTTCTGCAGGGGCG3').
Le fragment mutanéisé, purifié après une restriction BamHI-SalI, a été introduit dans pINA151 pour créer le plasmide pINA165. Par une sélétion EcoRV, on a généré le plasmide pINA166 qui présente le promoteur XPR2 et la séquence LEU2 (fragment de 5,3 kb de pINA62) dans pINA166. Enfin, le gène SUC2 de S. cerevisiae, provenant d'un fragment HindIII de 2 kb de pRB58 (réf. 18) a été inséré pour créer le plasmide final pINA169.

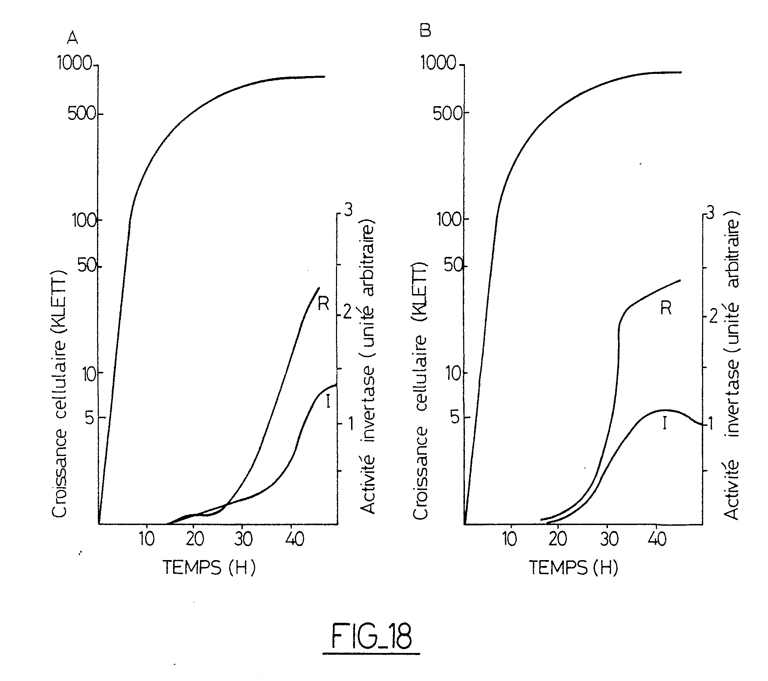

**Figure 18** : Comparaison de la production d'activité invertase au cours de la croissance par la souche JM58 possédant le gène SUC2 intégré (I) et par la souche P1 possédant le gène SUC2 sur le vecteur réplicatif pINA181 (R) à 28°C en milieu YPDm saccharose (A) et YPDm glucose (B).

**Figure 19** : Schéma de construction du plasmide pINA187 portant la fusion preproXPR2-IFN-alpha1. Le vecteur pLD67 contenant le gène pro-IFN-alpha1 codant pour l'interféron alpha-1 de porc a été décrit par La Bonnardière et Lefèvre (réf. 19). On l'a ouvert par les enzymes XbaI et ApaI puis ligaturé avec les oligonucléotides A (56-mère) et B (48-mère). Ceux-ci permettent de former un adaptateur XbaI-ApaI créant en phase avec la région "pré-pro" de la protéase un site Lys-Arg (souligné ci-dessous), suivi de la séquence codante pour l'interféron mature (pINA185).
A : CTAGATCTAAGCGATGTGACCTGCCCCAGACCCACTCCCTGGCCCACA CCAGGGCC
B : TAGATTCGTTACACTGGACGGGGTCTGGGTGAGGGACCGGGTGTGGTC
Le gène hybride est réalisé par l'insertion d'un fragment ClaI-XbaI de 1,9 kb qui reconstitue le promoteur XPR2 et la séquence codante pour le pré-pro de l'AEP suivie de la séquence codante pour l'interféron (pINA186). Le vecteur intégratif pINA187 possède le marqueur de sélection URA3 porté par un fragment EcoRI-HINDIII de 1,7 kb provenant de pINA156.

## EXEMPLE 1

## ISOLEMENT DE MUTANTS

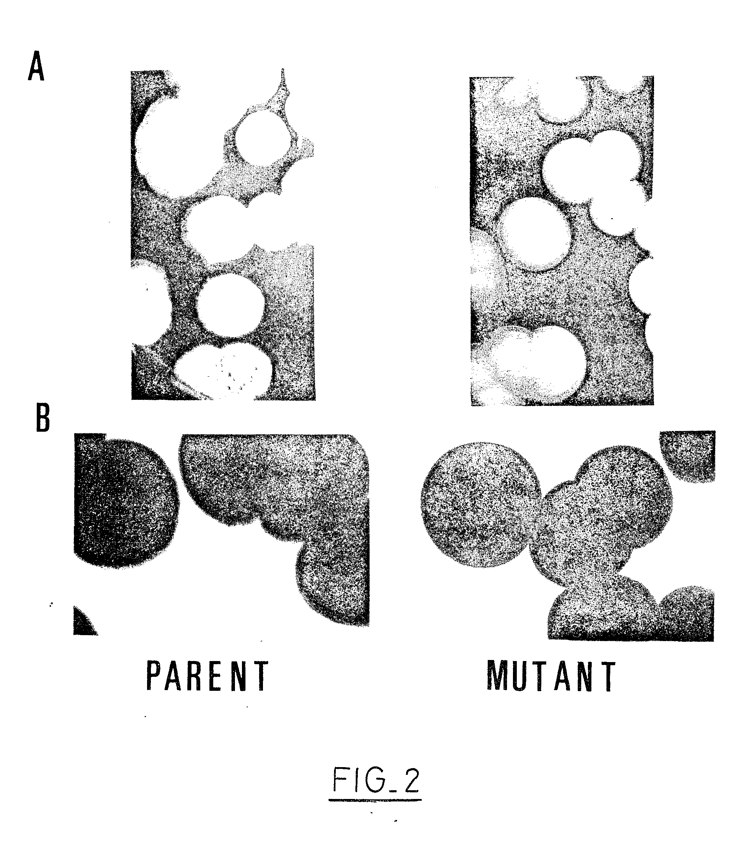

La formation de mycélium à l'échelle microscopique se traduit par une morphologie différente des colonies sur le plan macroscopique, à savoir : colonies dites "frisées" ou rugueuses (angl. rough), formant des peaux et entourées d'un chevelu à la surface de l'agar (fig. 2). Cette morphologie est plus nette quand les cellules ne sont pas trop nombreuses par boîte (moins de 100) et qu'elles sont sur un milieu riche avec forte concentration en glucose.

Pratiquement, on irradie une culture de la souche INAG33122 (MatB, leu2-35, xpr2, lys2-5, ade1) aux rayons U.V., à une dose permettant 60 % de survie. Après une phase d'expression en milieu complet, les cellules sont étalées (environ 500/boîte) sur milieu YPD$_{30}$ (yeast extract 1 %, peptone 1 %, glucose 30 g/l, agar 20 g/l) ou

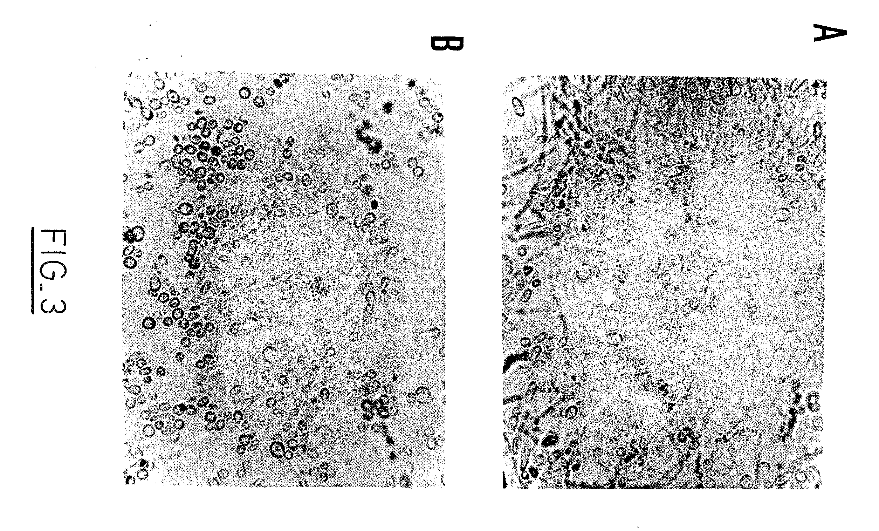

YPD10 (avec seulement 10 g/l de glucose). Après 8 jours d'incubation à 28°C, les colonies présentant une morphologie atypique sont repérées à la loupe binoculaire, puis purifiées. Leur phénotype est confirmé par des étalements à faible densité coloniale et observation microscopique (fig. 3). Finalement 7 mutants sont retenus sur 61 000 colonies initiales (fréquence 1.1 $10^{-4}$) et un seul présente un phénotype assez net et stable pour la suite des expériences. Ce phénotype est appelé Fil- et le génotype est noté fill-18. La souche porte le nom INAG33129 (MatB, leu2-35, xpr2, lys2-5, adel, fill-18).

EXEMPLE 2

CONSTRUCTION DE LA BANQUE

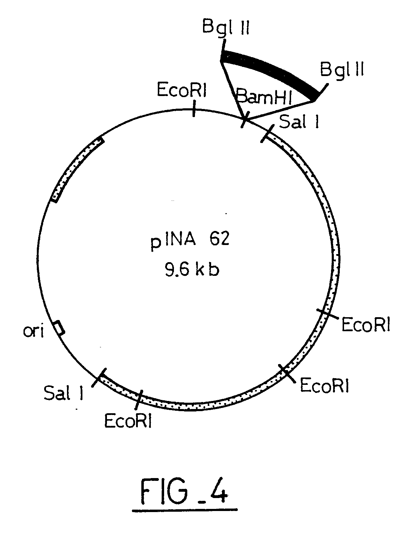

La souche réceptrice possédant la mutation (très stable) leu2-35 (en provenance de la souche DX465-7B de D.M. Ogrydziak, décrite dans le brevet cité en référence 8), on construit une banque dans le plasmide pINA62 (réf. 5) qui contient l'allèle LEU2 cloné au site SalI de pBR322 (fig. 4). L'ADN de la souche 15901-4 (MatB, ura2-21, lycl-5, LYS1-5) de Y. lipolytica a été digéré totalement par l'enzyme BglII et mélangé au plasmide pINA62 préalablement coupé par BamHI et déphosphorylé avec la phosphatase alcaline. La banque ne couvre pas entièrement le génome et est constituée de 4 ensembles (ou "pools") représentant un total de 7 000 clones dont 89 % sont dits hybrides (c'est-à-dire contiennent un fragment cloné). La taille moyenne des insertions, estimée sur une vingtaine de plasmides pris au hasard, est voisine de 5,2 kb. L'ADN de ces pools a été extrait et purifié sur gradient de chlorure de césium avec bromure d'éthidium.

EXEMPLE 3

SELECTION DES SEQUENCES ARS

Partant toujours de l'hypothèse selon laquelle les ars devraient être chez Y. lipolytica relativement instables, on transforme d'abord la souche INAG33129 avec l'ADN des pools et recherche ensuite parmi eux des clones instables. L'ADN des pools n'a pas été restreint préalablement, pour permettre une sélection directe de séquences à réplication extrachromosomique ; on vérifie cependant sur un petit échantillon que cet ADN coupé partiellement ou totalement par l'enzyme ApaI (site unique dans le plasmide de départ pINA62 et situé dans la séquence LEU2) est capable de transformer par intégration comme pINA62 (même si c'est à une fréquence plus faible que ce dernier). Les nombres de transformants sont indiqués dans le tableau I, en même temps que le nombre de clones testés et le pourcentage d'instables (qui est voisin de 10 %).

Tableau 1

Efficacité des différents pools d'ADN de la banque pour la transformation de Y. lipolytica et pour l'obtention de clones instables

| N° du pool | Taille du pool (1) | Efficacité de transformation (Tr/µg d'ADN) | | Nbre de clones (obtenus avec l'ADN non coupé) testés pour la stabilité | Nbre de clones instables | % de clones instables |
|---|---|---|---|---|---|---|
| | | ADN coupé par ApaI | ADN non coupé | | | |
| 1 | 2500 | 3700 | 15 | 85 | 10 | 12 |
| 2 | 2500 | 1500 | 300 | 16 | 12 | 16 |
| 3 | 1000 | 10500 | 70 | 44 | 1 | 2 |
| 4 | 1000 | N.T. | 10 | 13 | 0 | < 8 |
| total | | | | 218 | 23 | 11 |

(1) Nombre de clones bactériens

Pour tester l'instabilité des clones transformants, ceux-ci sont striés sur milieu riche et, après croissance, sont répliqués sur milieu minimum (Yeast Nitrogen Base sans sulfate d'ammonium 1,7 g/l, lysine source d'azote 1 g/l, adénine-HCl 100 mg/l, glucose 10 g/l, agar 20 g/l) et milieu minimum supplémenté en L-leucine (200 mg/l). Les clones instables sont ceux qui ont ségrégé significativement des colonies Leu⁻.

## EXEMPLE 4

## CARACTERISATION DES PLASMIDES ARS

Les colonies instables sont maintenues sur milieu sélectif, et leur ADN total est extrait par une méthode de minilysats qui a déjà été décrite (9). Cet ADN a subi une électrophorèse en gel d'agarose et a été ensuite transféré sur membrane de nylon (Biodyne, Pall France) par la méthode de Southern (10) et hybridé avec l'ADN du plasmide pINA62 marqué au $^{32}p$ par la technique de déplacement de coupure (Nick-Translation Kit, Amersham France).

Cette sonde radioactive permet de repérer l'allèle LEU2 chromosomique, sous forme d'une bande très diffuse, l'ADN n'étant pas restreint, et une bande extrachromosomique correspondant au plasmide transformant (pINA62+insertion). D'après les différences de migration des bandes plasmidiques (fig. 5), qui correspondent d'ailleurs souvent à des formes circulaires fermées (dites CCC pour "covalent closed circular"), on peut voir qu'il y a au moins 5 tailles de plasmides. Les bandes additionnelles que l'on voit dans certains puits pourraient correspondre soit à l'existence de plusieurs plasmides dans la même souche, soit à des formes circulaires ouvertes (OC pour "open circular") de la même molécule. Pour distinguer entre les deux hypothèses, on transforme la souche HB101 (hsdR⁻, hsdM⁻, recA13, supE44, lacZ4, leuB6, proA2, thi-1, $Sm^R$) d'E. coli avec l'ADN extrait des transformants en sélectionnant des colonies résistantes à l'ampicilline (le gène bactérien de résistance étant présent sur pINA62). Pour chacun des transformants de levure retenus, on obtient des colonies de E. coli desquelles le plasmide est extrait et analysé par enzymes de restriction. Il s'avère que chaque transformant de levure ne contient qu'un seul type de plasmide. Les cartes de restriction des 5 plasmides sélectionnés sont présentées sur la figure 6. L'analyse de ces cartes, ainsi que les résultats d'hybridation (données non décrites au présent brevet) réalisées entre des restrictions de ces plasmides et l'une des séquences ars (un fragment du plasmide pINA119) montrent que 3 des fragments ars clonés sont en fait homologues. Il s'agit du même fragment BglII-BglII de 4,5 kb, auquel se joignent éventuellement (dans le cas de pINA119 et pINA126) d'autres fragments lors de la ligation. En conclusion, 3 séquences ars différentes sont disponibles, portées par les plasmides pINA119, pINA123 et pINA124 et localisées sur des fragments d'ADN de 5,2 kb, 6,6 kb et 17,8 kb respectivement.

## EXEMPLE 5

## TRANSFORMATION DE Y. LIPOLYTICA AVEC LES SEQUENCES CLONEES

Pour montrer que les plasmides que l'on a sélectionnés comme molécules extrachromosomiques dans Y. lipolytica capables de transformer cette levure à haute fréquence, on utilise la souche mutante Fil- (INAG33129), mais également la souche Fil+ (INAG33122). Il s'avère que toutes deux sont transformables à haute fréquence par pINA119 (tableau II, expériences 1 et 2). On peut voir sur ces résultats que l'optimum de transformation de la souche INAG33129 par intégration (ici plasmides pINA62 ou pINA119 restreints par ApaI) ne correspond pas à l'optimum de transformation réplicative obtenu avec pINA119 non restreint (comparer l'expérience 3 pour le premier cas avec les expériences 1 et 2 pour le second). Le déterminisme biologique de cet optimum est encore inconnu, ce qui explique la très nette fluctuation quantitative des résultats d'une expérience à l'autre. Cependant, cette fluctuation ne met pas en cause le fait que le plasmide pINA119 transforme toujours de 20 à 100 fois plus que le plasmide pINA62 intact de départ, ce qui témoigne bien de la présence de l'ars.

Dans l'expérience n° 3, les plasmides pINA125 et pINA126, qui s'avèrent porter un fragment commun avec pINA119, transforment à peu près à la même fréquence la souche INAG33129 (pINA125 qui est un peu plus petit transforme mieux). On peut considérer que pINA123 et pINA124 sont à peu près aussi efficaces, d'autant plus que pINA124 a une taille presque double de pINA119.

Par ailleurs, l'ensemble des données du tableau II révèle que la souche Fil+ est également transformable par les plasmides ars, quoiqu'à moins haute fréquence que le mutant Fil- dans cette expérience. Ces plasmides transforment au moins 100 fois mieux la souche INAG33122 que pINA62 non restreint. Ce résultat paraît très surprenant du fait de l'hypothèse initiale mettant en cause le mode de division cellulaire dans la sélection des ars. Il est donc nécessaire de regarder si un plasmide ars se

Tableau II

Efficacités de transformation de divers plasmides sur les deux souches Fil+ et Fil-, exprimées en nombre de transformants par µg d'ADN

| Expérience | Souche | Phénotype | pINA62 | | pINA119 | | A.D.N. pINA171 | pINA123 | pINA124 | pINA125 | pINA126 |
|---|---|---|---|---|---|---|---|---|---|---|---|
| | | | NR | R | NR | R | NR | NR | NR | NR | NR |
| 1 | INAG33129 | Fil- | NT | 20000 | 15000 | 16000 | NT | NT | NT | NT | NT |
| 2 | INAG33129 | Fil- | NT | 8900 | 47000 | 15000 | NT | NT | NT | NT | NT |
| | INAG33122 | Fil+ | NT | 80000 | 55000 | 25000 | | | | | |
| 3 | INAG33129 | Fil- | 800 | 88000 | 6000 | NT | 33000 | 6900 | 2100 | 15000 | 6100 |
| | INAG33122 | Fil+ | 0 | 12000 | 600 | NT | 1000 | 250 | 375 | 750 | 500 |

NR : non restreint

R : restreint par ApaI

NT : non déterminé

maintient également de façon extrachromosomique dans la souche Fil-. Une expérience d'hybridation après transfert de Southern analogue à celle décrite à l'exemple 4 montre que tel est bien le cas dans des transformants Fil+ obtenus par pINA119 (données non présentées). Pour caractériser davantage cette situation inattendue, on étudie la stabilité des transformants.

## EXEMPLE 6

## STABILITE DES SEQUENCES ARS

La stabilité des 5 plasmides pINA119 et pINA123 à pINA126 est estimée après 5 à 10 générations en milieu sélectif (sans leucine) liquide et étalement parallèle sur milieu minimum et milieu supplémenté en leucine. Le rapport du nombre de colonies comptées sur l'un et l'autre milieu donne le pourcentage de prototrophes tel que présenté sur le tableau III A. Ce pourcentage varie de 65 % à 84 % et il n'y a pas de différence statistiquement significative entre les stabilités des différentes séquences ars dans la souche INAG33129. En revanche, il est remarquable que ce pourcentage soit si élevé, contrairement au comportement des séquences ars chez S. cerevisiae (2).

Pour étudier en détail cette stabilité, on s'intéresse plus particulièrement à la séquence ars présente sur pINA119. Des transformants obtenus soit dans la souche Fil- (INAG33129), soit dans la souche Fil+ (INAG33122) sont cultivés pendant environ 10 générations en milieux sélectif et non sélectif et le pourcentage d'auxotrophes estimé comme précédemment, dans chaque cas. Les résultats du tableau III B confirment la relative stabilité de l'ars en milieu sélectif dans INAG33129, mais semblent indiquer une stabilité encore plus grande dans la souche Fil+. Pour confirmer ce résultat, on transforme une souche leu2-35 dont le phénotype de croissance mycélienne est encore plus marqué (souche dite Fil++) et on constate une stabilité très haute, voire absolue dans cette souche. On vérifie (données non présentées) que l'ADN de pINA119 est toujours visible comme une bande extrachromosomique par hybridation. La stabilité en milieu non sélectif, bien que plus faible qu'en milieu sélectif dans chaque cas, suit également la même progression en fonction du phénotype Fil-/Fil+.

Tableau III

A) Stabilité après 5 générations en milieu sélectif dans la souche INAG33129 (Fil-)

| ADN | pINA119 | pINA123 | pINA124 | pINA125 | pINA126 |
|---|---|---|---|---|---|
| % prototrophes | 65 | 72 | 84 | 79 | 69 |

B) Stabilité du plasmide pINA119 après 10 générations sur divers milieux et dans plusieurs souches

| Souche | Phénotype | % de prototrophes sur milieu sélectif | % de prototrophes sur milieu non sélectif |
|---|---|---|---|
| INAG33129 | Fil- | 60-70 | 10-15 |
| INAG33122 | Fil+ | 70-80 | 60 |
| MLII | Fil++ | 85-100 | 80-90 |

On pense donc que, contrairement à l'hypothèse initiale, l'échec dans l'isolement des ars n'est pas dû à une trop grande instabilité de ces séquences qui empêcherait leur propagation dans les mycéliums, mais plutôt au fait qu'on ne pouvait jamais les retrouver en cherchant des clones instables. En effet, dans les souches Fil+, ces séquences semblent aussi stables que des intégrations.

Dans ces conditions, il est nécessaire de démontrer que les plasmides restent toujours extrachromosomiques et que la stabilité n'est pas due à l'existence d'une copie intégrée. Pour cela, on cultive 30 transformants de INAG33122 et 30 transformants de INAG33129 pendant 50 générations sur milieu sélectif pour voir si on trouve un changement dans la stabilité de ces clones. Le protocole détaillé de l'expérience est fourni au schéma ci-après et au tableau IV :

50 générations sur milieu minimum sans leucine
2 jours sur milieu minimum - leucine
Etalements parallèles sur milieu minimum et milieu minimum - leucine
Stabilité initiale
2 jours sur milieu minimum - leucine
Etalements parallèles sur milieu minimum et milieu minimum + leucine
Stabilité finale

Tableau IV

Effet d'un passage en milieu sélectif pendant 50 générations sur la stabilité

(Les résultats sont exprimés en nombre de clones se rangeant dans une catégorie donnée de ségrégations (% de prototrophes). L'expérience porte sur 30 clones de la souche Fil+ et 30 clones de la souche Fil-.

| Souche | Stabilité | % de prototrophes | | |
|---|---|---|---|---|
| | | <40 % | 40-60 % | > 60 % |
| Fil+ | initiale | 0 | 11 | 19 |
| | finale | 5 | 13 | 12 |
| Fil- | initiale | 4 | 18 | 8 |
| | finale | 12 | 11 | 7 |

L'estimation qualitative en pourcentage de prototrophes obtenus par un passage de deux jours en milieu non sélectif, soit avant, soit après les 50 générations, montre (tableau IV) qu'il n'y a pas de translation vers un profil plus stable, qu'il s'agisse de la souche Fil+ ou de la souche Fil-. Pour démontrer l'absence d'intégration, on extrait l'ADN de transformants Fil+ et Fil- avant et après qu'ils aient subi les 50 générations et on les restreint par les enzymes ApaI et BamHI. Les ADN restreint et non restreint sont transférés sur Biodyne après migration sur gel d'agarose (0,7 % en tampon Tris 89 mM, acide borique 89 mM, EDTA 2,5 mM, pH 8,3) et hybridés à une sonde radioactive pBR322. Le résultat (non présenté) montre clairement la présence des bandes plasmidiques et l'absence de bandes dues à une intégration.

## EXEMPLE 7

### ANALYSE FINE DE L'INSERTION PRESENTE DANS pINA119

Chez S. cerevisiae, il est connu que certaines séquences ars sont répetées dans le génome (en particulier celles qui se trouvent près des extrémités télomériques) alors que d'autres sont présentes à un seul exemplaire (1,2,11). On regarde ce qu'il en est pour la séquence ars de pINA119. Pour ce faire, on purifie un fragment interne PstI-EcoRI de 4,6 kb (fig. 7) qui couvre la quasi-totalité de l'insertion et on l'utilise comme sonde contre des transferts de restriction d'ADN plasmidique et génomique ayant migré en parallèle sur gel d'agarose. Les résultats, analysés à la lumière de la carte de restriction de pINA119 déjà présentée à la figure 6 (voir aussi fig. 7), montrent qu'il s'agit probablement d'une séquence unique (à moins d'une répétition exacte en tandem d'un fragment BglII-BglII). La bande interne BamHI de 2,2 kb se retrouve à la même position dans le génome, de même que la bande interne HindIII de 2,3 kb. Le fragment BglII revélé dans le génome par la sonde correspond bien à une seule bande de 4,5 kb qui est identique au fragment cloné qui porte l'ars dans pINA119 (en fait dans pINA119 il y a deux fragments BglII qui ont été associés lors de la ligation, il s'agit ici du plus grand des deux, qui est homologue aux insertions de pINA125 et pINA126).

Cette taille étant certainement (par comparaison avec les ars décrites chez S. cerevisiae (1,2,11) largement supérieure à celle de la séquence minimale requise pour la fonction ars, on procède à des expériences de délétions et de reclonages pour localiser plus précisément l'ars dans l'insertion de pINA119. La stratégie suivie est décrite sur la figure 7, d'où l'on peut conclure que l'ars doit être portée par le fragment interne BamHI-BamHI de 2,2 kb qui est présent dans pINA171. Ce plasmide est capable de transformer à haute fréquence aussi bien la souche Fil- que la Fil- (tableau II). Il est capable de s'y maintenir de façon extrachromosomique (donnée non présentée) et sa stabilité y est analogue à celle de pINA119 (environ 65 % de prototrophes après 5 générations en milieu sélectif dans la souche INAG33129 et 81 % dans INAG33122). Ce plasmide a servi de base à des constructions ultérieures en vue d'amplifications de gènes.

Par ailleurs, le fragment BamHI-BamHI a été recloné dans un vecteur "Bluescript M13" qui est un hybride entre le phage M13 et le plasmide bactérien de type colE1. Ce vecteur porte un multisite de clonage qui a permis, d'une part, d'insérer l'ars au site BamHI et, d'autre part, la séquence codante minimale de 3 kb de LEU2 comprise entre un site SalI et un site ApaI (que l'on a cloné entre XhoI et ApaI du multisite). Ce vecteur de 8,2 kb est capable de transformer Y. lipolytica INAG33129 à haute fréquence et a servi de base à une nouvelle série de délétions et de reclonages liés à l'établissement d'une carte fine de l'ars décrite sur la figure 8.

## EXEMPLE 8

### Expression et amplification de gènes grâce à la séquence ars

Afin de démontrer l'utilité de l'ars présente sur pINA119 pour amplifier des gènes, on réalise la construction plasmidique suivante : le gène lacZ d'E. coli codant pour la béta-galactosidase pouvant être exprimé chez Y. lipolytica sous le contrôle du promoteur de LEU2 (5), on clone le fragment ars BamHI-BamHI de 2,2 kb dans un site BglII qui est présent sur pINA98 (fig. 9), plasmide intégratif portant LEU2 et la construction d'expression de lacZ. Le nouveau plasmide pINA135 est donc réplicatif et est introduit dans INAG33122 et INAG33129.

L'expression de la béta-galactosidase est repérée d'abord sur boîte de milieu minimum contenant le substrat chromogène X-gal. après toluénisation pendant une minute, les boîtes sont mises à incuber à 37° C et on observe que l'intensité de la coloration developpée par les transformants réplicatifs est bien supérieure à celle du transformant monocopie. Pour quantifier cet effet, on réalise des extraits bruts des transformants et on dose l'activité béta-galactosidase selon le protocole de Miller (12) et on la rapporte à la quantité de protéines estimée selon la méthode de Bradford (13). Cette activité spécifique de la béta-galactosidase est corrigée par le pourcentage de cellules prototrophes dans la culture au moment où les cellules sont broyées. Ce pourcentage est estimé par des étalements parallèles en milieux sélectif et non sélectif. Les résultats d'activité présentés sur le tableau V montrent que l'amplification obtenue avec l'ars varie d'un facteur 2 à 9 et est voisin dans les souches Fil+ ou Fil-

Tableau V

| Souche | Plasmide | Densité cellulaire | Activité spécifique | % de prototrophes | Activité spécifique corrigée | Amplification |
|---|---|---|---|---|---|---|
| Fil+ | pINA171 | 2,3.10$^{7}$ | 1,6 | 81 | 1,6 | 0 |
| Fil- | pINA171 | 5,0.10$^{6}$ | 43 | 64 | 43 | 0 |
| Fil+ | pINA98 | 3,7.10$^{7}$ | 218 | 100 | 218 } 390 | 1 |
| | | 1,5.10$^{7}$ | 562 | 100 | 562 | |
| Fil- | pINA135 | 5,1.10$^{7}$ | 2141 | 100 | 2141 | 5,5 |
| | | 8,6.10$^{7}$ | 1101 | 100 | 1101 | 2,8 |
| | | 1,0.10$^{8}$ | 2447 | 70 | 3496 | 9 |
| Fil- | pINA135 | 2,7.10$^{7}$ | 690 | 83 | 831 | 2,1 |
| | | 6,5.10$^{6}$ | 1744 | 87 | 2001 | 5,1 |

Mesure de l'activité spécifique de la béta-galactosidase dans des extraits bruts. La densité cellulaire au moment de l'extraction est exprimée en nombre de cellules par ml ; l'activité spécifique est en unités internationales (une unité est définie comme la quantité d'enzyme qui hydrolyse $10^{-9}$ moles par min. d'orthonitrophénol-galactoside à 37°C) et elle est ensuite corrigée pour tenir compte du pourcentage de prototrophes dans la culture. Un niveau de 1 est arbitrairement attribué à la moyenne de deux mesures obtenues avec des clones transformés par pINA98 (plasmide intégratif). Le niveau zéro correspond aux souches transformées par pINA171 (LEU2+ars sans lacZ). Les différentes lignes de résultats avec pINA135 (plasmide réplicatif décrit figure 9) dans les souches Fil+ ou Fil-correspondent à des transformants indépendants.

## EXEMPLE 9

## ISOLEMENT DE SEQUENCES ARS

Afin de prouver que l'isolement de séquences ars efficaces pour Y. lipolytica n'était pas dépendant de la souche Fil$^{-}$ (mutant morphologique), nous avions déjà montré que les plasmides portant une séquence ars pouvaient se répliquer dans une souche Fil$^{+}$. Nous avons maintenant cherché à montrer que l'on pouvait également trouver les ars en transformant directement une souche Fil$^{+}$ avec la banque de gènes. Tout d'abord la même banque a été utilisée (le "pool n°2" de celle-ci) et parmi les transformants Leu$^{+}$ nous avons sélectionné des instables (voir tableau VI). Les plasmides qu'ils hébergent ont été isolés par transformation de E.Coli avec des minipréparations d'ADN total des transformants de levure. Nous avons trouvé des clones portant ars18, précédemment isolée dans la souche Fil$^{-}$ à partir de ce même "pool". Cependant, ces clones n'étaient pas strictement identiques au plasmide pINA119 décrit initialement, du fait que lors de la construction de la banque (fragments provenant d'une restriction totale d'ADN d'une souche sauvage par BglII et insérés dans le site BamHI de pINA62), il avait pu y avoir religation de plusieurs fragments BglII dans un même plasmide.

Ignorant si la séquence AGATCT (site BglII) pouvait faire partie d'un éventuel consensus d'ars pour Y.lipolytica, nous avons décidé d'utiliser également une autre banque construite par digestion partielle du génôme d'une souche sauvage (W29) par Sau3A, sélection des fragments de taille d'environ 10-15kb, et clônage dans le site BamHI du même plasmide pINA62. Cette banque, décrite dans la référence 14, est représentative du génôme Y.lipolytica et a d'ailleurs permis l'obtention, par intégration dirigée dans le chromosome, de trois gènes de Y.lipolytica. Nous avons utilisé l'ADN non restreint de cette banque pour transformer la souche Fil$^+$ et la souche Fil$^-$. Dans ce cas, nous avons de nouveau repéré les clones instables pour le phénotype Leu$^+$. Pour cela on cultive les transformants en deux passages successifs sur un milieu minimum solide supplémenté en leucine, puis on strie sur ce même milieu pour obtenir des colonies isolées que l'on trie par répliques sur milieux minimums avec et sans leucine. Nous avons ainsi obtenu deux nouveaux transformants ars, en cours d'analyse. Les séquences correspondantes sont notées ars* dans le tableau VI, où est résumé l'ensemble des résultats des transformations réalisées avec les deux banques d'ADN.

TABLEAU VI

| Banque | Expérience N° | Souche | Fréquence transf. | Clones testés | Nombre Instables | % Inst. | ars trouvées | % ars différentes |
|---|---|---|---|---|---|---|---|---|
| BglII in pINA62 | 1 | Fil$^-$ | Pool | | | | | |
| | | | 1 15T/µg | 85 | 10 | 11.7 | ars68 | |
| | | | 2 300T/µg | 76 | 12 | 15.8 | ars77 | |
| | | | 3 70T/µg | 44 | 1 | 2.2 | ars18 | |
| | | | 4 10T/µg | 13 | 0 | <7.6 | - | |
| | | | Bilan 100T/µg | 218 | 23 | 10.6 | | 1,4% |
| | 2 | Fil$^-$ | Pool 2 200T/µg | 25 | 3 | 12.0 | ars18 | 4.0% |
| | 3 | Fil$^+$ | 2 100T/µg | 181 | 6 | 3.3 | ars18 | 0,5% |
| Sau3A (partiel) in pINA62 | 4 | Fil$^-$ | 200T/µg | 44 | 1 | 2.2 | ars* | 2,2% |
| | | Fil$^+$ | 650T/µg | 131 | 1 | 0.8 | ars* | 0,8% |

TABLEAU VI : Obtention de séquences ars dans des souches Fil$^+$ ou Fil$^-$ transformées par différentes banques génomiques. Le nom des séquences ars trouvées est indiqué (ars* pour les séquences en cours de caractérisation). La dernière colonne indique le rapport constitué par le nombre d'ars indépendantes trouvées sur le nombre de clones testés, pour une expérience donnée.

Si l'on tient compte uniquement des séquences ars réellement différentes isolées, on voit que celles-ci sont sélectionnées à une fréquence de l'ordre de 2,5 % dans un contexte Fil$^-$, et de l'ordre de 0,6 % dans un contexte Fil$^+$, quel que soit le type de banque utilisé (tableau VII)

| Souche \ Banque | BglII total | Sau3A partiel |
|---|---|---|
| $Fil^{-}$ | 2.7% | 2.2% |
| $Fil^{+}$ | 0.5% | 0.8% |

Pourcentage de clones $Leu^{+}$ possédant des séquences ars différentes parmi les transformants de Y. lipolytica obtenus avec les deux banques génomiques, sur l'ensemble des 4 expériences présentées au tableau VI.

Ces chiffres sont bien plus faibles que ceux connus ches S. cerevisiae, et montrent que seules quelques séquences sont capables de supporter la réplication autonome chez Y.lipolytica. Cependant ces résultats démontrent nettement la généralité du procédé d'isolement de séquences ars dans n'importe quelle souche de cette espèce.

Il est par ailleurs clair pour l'homme de l'art que le procédé que nous avons utilisé pour isoler des séquences ars efficaces chez Y. lipolitica pourrait être répété avec un ADN d'origine quelconque. A titre d'exemple, nous avons pu montrer que l'ADN du bactériophage contenait (entre les positions 44972 bp et 48502bp, Roberts R.J., 1987, Nucleic Acid Research, 15, r189-r217) une telle séquence efficace.

## EXEMPLE 10

## REDUCTION ET CARACTERISATION DES SEQUENCES ARS

En vue de mieux caractériser les séquences décrites précédemment, nous avons réalisé des sous-clonages et des délétions des divers plasmides pINA171, pINA123, et pINA124 (voir figures 6 et 7). Les constructions faites à partir des deux derniers plasmides sont décrites sur la figure 10, où l'on voit qu'il est possible de localiser la partie efficace des deux ars68 et ars77 sur des fragments de 2,7 kb et 2,4 kb respectivement, ce qui représente une très forte réduction de la taille d'ADN nécessaire pour bâtir des vecteurs ars. En effet, les insertions d'ADN de Y. lipolytica étaient initialement de 6,6 kb et 17,8 kb dans les plasmides pINA123 et pINA124 respectivement.

Par ailleurs, le même type de stratégie nous a conduit à analyser la séquence ars18. Les constructions plasmidiques ont été réalisées à partir du plasmide pINA171 décrit précédemment, ou à partir de pINA147 qui est le minivecteur de la figure 8A. Ces deux vecteurs contiennent le même fragment BamHI de 2,2 kb qui porte l'activité ars, le même gène marqueur LEU2 (un fragment SalI-ApaI de 2,0 kb dans le cas de pINA147, et dans le cas de pINA171, le fragment SalI-SalI de 5,3 kb qui se trouve aussi dans le plasmide pINA62 (figure 4). Il diffèrent par la partie bactérienne qui est constituée de Bluescript (Stratagène, 3770 Tansy Street, San Diego, CA 92121) pour pINA147 et de pBR322 pour INA171.

L'ensemble des constructions plasmidiques a été réalisé par des délétions ou des reclonages faisant usage des sites de restriction indiqués sur la figure 11, sauf dans le cas de pINA211. Ce dernier plasmide a été obtenu de la manière suivante, inspirée des descriptions de délétions in vivo observées chez S.cerevisiae (15, 16) : on a linéarisé le plasmide pINA147 par l'enzyme ClaI, dont le site se trouve dans la partie Bluescript quelques bases en amont de l'ars ; on a transformé la levure avec le plasmide ainsi linéarisé ; on a repéré les clones instables pour le phénotype $Leu^{+}$, et on en a extrait les plasmides qu'ils contenaient. Ce plasmide s'est avéré contenir une délétion d'environ 70 paires de bases réalisée in vivo par la levure Y. lipolytica avant qu'elle ne religature ce plasmide sur lui-même pour qu'il se propage ensuite à l'état de molécule circulaire.

Ainsi, qu'il est présenté sur la figure 11, nous avons pu mettre en évidence que les séquences responsables de la transformation à haute fréquence et de la réplication extrachromosomique peuvent être physiquement localisées sur deux régions adjacentes. Pour conserver les deux fonctions, nous avons décidé d'utiliser le fragment de 1,3 kb présent dans le plasmide pINA176. Ce fragment ars est délimité par deux sites BamHI, car la ligation du site Sau3A de droite au site BamHI du vecteur a reconstitué un site BamHI. Ce fragment a donc pu être inséré dans d'autres vecteurs basé sur pBR322, soit dans le site BamHI présent dans le gène codant pour la résistance à la tétracycline (pINA232), soit dans le site BglII présent dans la partie terminale de la séquence de LEU2 (pINA237 et pINA237') ou soit enfin au site BglII d'un multisite de clonage inséré au site PvuII du vecteur (pINA240 et pINA240'). Les vecteurs pINA237, pINA240, et pINA240', et pINA240' sont particulièrement pratiques car l'insertion de l'ars ayant eu lieu à un site BglII, les sites BamHI de l'insertion ont disparu.

Ces vecteurs comportent dès lors :

- la séquence de 2 kb permettant l'expression du gène LEU2 ;
- la séquence de l'ars18 réduite à 1,3 kb ;
- les deux gènes de résistance aux antibiotiques ampicilline et tétracycline ;
-un site unique BamHI permettant le clonage (en particulier de fragments provenant d'une digestion partielle par Sau3A pour la construction d'une banque génomique sur un tel plasmide réplicatif) avec repérage des clones hybrides comme sensibles à la tétracycline.

Les cartes des plasmides pINA176, pINA232, pINA237, pINA237′, pINA240 et pINA240′ sont décrites sur la Figure 12. Tous ont été testés pour leur capacité à transformer la souche Fil+ INAG33122, et tous peuvent permettre d'obtenir plus de 5000 transformants par µg d'ADN non restreint.

Par ailleurs, le fragment de 1,3 kb de l'ars18 a été caractérisé plus avant par sa séquence nucléotidique établie par la méthode de Sanger aux didéoxynucléotides, sur les deux brins. La Figure 13 montre cette séquence de 1305 nucléotides, caractérisée par une richesse en AT (67,5 %) et par un profil complexe de répétition internes de 10 à 13 bases, soit de façon directe, soit de façon inverse, et dont les plus significatives sont soulignées. Comme la plupart d'entre elles se trouvent dans la partie de la séquence dont nous avons montré plus haut qu'elle est importante pour la réplication autonome, on peut se demander si elles jouent un rôle dans cette fonction.

## EXPRESSION ET AMPLIFICATION DE GENES GRACE A LA SEQUENCE ARS

Nous avons préalablement prouvé l'utilisé de l'ars18 présente sur pINA119 pour amplifier le gène lacZ d'E.coli. Nous démontrons ici que cette séquence ars18 permet l'amplification du gène homologue XPR2 de Y.lipolytica codant pour la protéase alcaline extracellulaire ou AEP (17), des gènes hétérologues SUC2 de S.cerevisiae codant pour l'invertase (18), du gène pro-INF-α1 de porc codant pour l'interféron α1 (19), et du gène pro-REN codant pour la chymosine bovine (19). L'amplification de ces gènes a permis d'augmenter la production et la sécrétion des protéines correspondantes. L'ensemble des plasmides et souches utilisés est décrit sur la figure 14 et dans le tableau VIII.

TABLEAU VIII

| PROTEINES SECRETEES | PLASMIDES | CARACTE-RISTIQUES | GENES | MAR-QUEURS | SOUCHES TRANSFOR-MEES | SOUCHES RESUL-TANTES |
|---|---|---|---|---|---|---|
| | -- | CHROMO-SOMIQUE | XPR2 | | | JM12 |
| AEP | pINA157 | DISRUPTION | xpr2::LYS5 | LYS5 | JM12 | JM23 |
| | pINA136 | REPLICATIF | XPR2 | LEU2 | JM23 | JM69,JM70 |
| | pINA169 | INTEGRATIF | SUC2 | LEU2 | JM23 | JM55 |
| INVERTASE | pINA181 | REPLICATIF | SUC2 | LEU2 | JM23 | P1 |
| INTERFERON | pINA187 | INTEGRATIF | proINFα | URA3 | JM23 | CM77 |
| | pINA250 | REPLICATIF | proINFα | LEU2 | JM23 | JM85-JM87 |
| CHYMOSINE | pLX34 | INTEGRATIF | proREN | LEU2 | DL118 | L249 |
| | - | - | - | - | INAG33122 | LX2-1 |
| | pINA184 | REPLICATIF | proREN | LEU2 | DL118 | TF1,2,4,5 |
| | - | - | - | - | INAG33122 | 2-36 |

Nom des vecteurs intégratifs et réplicatifs utilisés pour l'amplification du gène homologue XPR2 et des gènes hétérologues SUC2, pro-INF 1 et pro-REN. On trouve aussi dans les deux dernières colonnes les noms des souches receveuses et des transformants retenus pour les dosages des diverses activités.

## EXEMPLE 11

## EXPRESSION ET AMPLIFICATION DU GENE XPR2

Le gène XPR2 de Y.lipolytica codant pour la protéase alcaline extracellulaire (AEP) a été inséré dans un plasmide réplicatif puis introduit dans Y. lipolytica. Nous avons comparé la production de protéase alcaline extracellulaire sécrétée par les souches suivantes de notre collection : - JM12 (MatB, leu2-35, lys5-12, ura3-18, XPR2 a été disrupée à l'aide du plasmide pINA157 décrit sur la figure 14. Cette disruption est obtenue par transformation de la souche JM12 avec le plasmide pINA157 préalablement restreint par les enzymes de restriction MluI et EcoRI. Selon le principe présenté sur la figure 15, nous avons obtenu par double crossing over le remplacement du gène XPR2 par le gène LYS5. La souche résultante JM23 est donc isogénique à la souche JM12 à l'exception du marqueur LYS5 et du gène disrupté XPR2. Cette souche, ne produisant plus de protéase alcaline extracellulaire, est une bonne réceptrice pour la production de protéines. Nous avons transformé cette souche isogénique à JM12 par le plasmide réplicatif pINA136. JM69 et JM70 qui correspondent à la souche JM23 transformée par le plasmide réplicatif pINA136.

La comparaison de la quantité d'AEP sécrétée par les souches JM12, JM23, JM69 et JM70 est présentée dans le Tableau IX, qui montre que l'on peut obtenir avec l'ars18 une amplification corrigée d'un facteur 3,4 à 5,6. Ce facteur d'amplification corrigée tient compte du pourcentage de cellules Leu$^+$ dans la culture au moment du dosage, ainsi qu'il a déjà été expliqué précédemment pour le dosage de la β -galactosidase.

TABLEAU IX

| Souche | Plasmide | Densité cell. (Klett) | Activité | % LEU+ | Amplification corrigée |
|---|---|---|---|---|---|
| JM23 | pINA157 in JM12 | 475 | 0 | 100 | - |
| JM12 | pas de plasmide | - | 100 | 100 | 1 |
| JM69 | pINA136 in JM23 | - | 274 | 80 | 3.4 |
| JM70 | pINA136 in JM23 | - | 446 | 80 | 5.6 |

Comparaison de la production d'activité protéasique sécrété par la souche disruptée JM23, la souche JM12 qui possède une copie chromosomique du gène XPR2 et les transformants JM69 et JM70 qui présentent le gène XPR2 sur un plasmide réplicatif. La culture est réalisée à 28°C en milieu GPP (Glycérol 6,7 g/l, Yeast Nitrogen Base sans sulfate d'ammonium 1,7 g/l, glutamate 1g/l, protéose peptone 1,7 g/l, tampon phosphate 50 mM pH 6,8). La culture a été ensemensée à 10 unités Klett et le surnageant a été prélevé 23 heures après l'ensemensement. L'activité protéasique est déterminé selon la méthode de Donnelly et Coll. (27) et est exprimée ici en unités arbitraires. Le pourcentage de cellules Leu$^+$ est déterminée par étalement d'un aliquot de la culture, sur milieu supplémenté avec de la leucine puis répliqués sur milieux avec et sans leucine. L'amplification corrigée est calculée avec les activités corrigées par le pourcentage de cellules Leu$^+$.

Le promoteur du gène XPR2 présente une régulation complexe (Ogrydziak et coll. (21)). La cinétique et les taux de production dépendent du milieu et des conditions de culture. Nous avons donc comparé les cinétiques de production de la souche JM12 et la souche JM70 en milieu non inducteur YNB (Yeast Nitrogen Base sans sulfate d'ammonium 1,7 g/l, glutamate de sodium 1 g/l, glucose 10 g/l, tampon phosphate pH 6,8 50 mM) et dans deux milieux inducteurs $YNB_{pp}$ (YNB additionné de 1,7 g/l de protéose peptone) et YPDm (extrait de levure 1 g/l, protéose peptone 50 g/l, glucose 10 g/l). L'activité protéasique a été mesurée avec l'azocoll (Sigma A8143) comme substrat (10 mg/ml en Tris 50 mM, pH8) en mesurant l'augmentation d'absorbance à 520 nm après une incubation de 30 minutes à 30°C.

Les résultats sont présentés sur la figure 16 et le tableau 10.

TABLEAU X

| SOUCHES | PLASMIDES | CARACTERISTIQUES | MILIEU | ACTIVITE AEP | % LEU+ | AMPLIFICA-TION CORRIGEE |
|---|---|---|---|---|---|---|
| JM12 | - | CHROMO-SOMIQUE | YNB | 0.08 | 100% | 1 |
| JM12 | - | CHROMOSOMIQUE | YNBpp | 4.2 | 100% | 1 |
| JM12 | - | CHROMOSOMIQUE | YPDm | 12.2 | 100% | 1 |
| JM70 | pINA136 | REPLICATIF | YNB | 3.4 | 81.6% | 52 |
| JM70 | pINA136 | REPLICATIF | YNBpp | 12 | 84.6% | 3.4 |
| JM70 | pINA136 | REPLICATIF | YPDm | 60.2 | 83.6% | 5.9 |

Valeurs d'activité obtenues 40 heures après l'inoculation. L'activité protéasique est en unité arbitraire. Le facteur d'amplification obtenu au temps 40 h après l'inoculation varie entre 3,4 et 5,9 pour les cultures réalisées sur milieu inducteur. En revanche, en milieu répresseur (YNB), il y a probablement échappement à la régulation dans le cas des transformants réplicatifs, ce qui explique une amplification d'un facteur 50. Ce phénomène encore inexpliqué devrait permettre d'identifier l'élément régulateur et suggère donc qu'il existe d'autres possibilités d'amplification de l'expression encore mal exploitées.

## EXEMPLE 12

## EXPRESSION ET AMPLIFICATION DU GENE SUC2

Le gène SUC2 de S. cerevisiae codant pour l'invertase (18) a été mis sous le contrôle du promoteur XPR2 et fusionné à la séquence signal de la protéase alcaline extracellulaire (voir figure 17). Nous avons comparé la production d'invertase dans deux souches :

- JM55 qui correspond à la souche JM23 transformée par le plasmide intégratif pINA169 intégré au site XPR2. L'intégration est dirigée au site XPR2 car le plasmide pINA169 a été préalablement restreint par l'enzyme de restriction NheI, dont le site unique de coupure se trouve dans la région XPR2.
- P1 qui correspond à la souche JM23 transformée par le plasmide réplicatif pINA181.

L'activité invertase a été mesurée par la méthode de Werner et al. (22). L'amplification de la production d'invertase a été mesurée en milieu inducteur YPDm dont la source de carbone est le saccharose (10 g/l) ou le glucose (10 g/l). Les résultats sont illustrés sur la figure 18. Nous avons obtenu, après 40 heures de culture, un facteur d'amplification non corrigé (c'est-à-dire en considérant que toutes les cellules sont productrices) de 2 à 2,9 en glucose ou en saccharose. Nous pouvons noter que cette construction permet à Y. lipolytica de pousser sur un milieu contenant le saccharose comme source de carbone ce qui représente donc un nouveau substrat pour cet organisme.

## EXEMPLE 13

## EXPRESSION ET AMPLIFICATION DU GENE proINF-α1

Le gène pro-INF-α1 du porc codant pour l'interféron-α1 à activité antivirale a été mis sous le contrôle du promoteur XPR2 et fusionné à la partie "pré-pro" de l'AEP (pour la construction voir la figure 19). Nous avons comparé la production d'interféron produit par la souche présentant une copie du gène intégré au site XPR2 (JM85 à JM87) présentant le gène sur un plasmide réplicatif (pINA250) :

- la souche JM77 correspond à la souche JM23 transformée par le plasmide pINA187 préalablement restreint par l'enzyme de restriction MluI, dont le site unique de coupure se trouve dans la région XPR2 ;
- les souches JM85, JM86 et JM87 correspondent à la souche JM23 transformée par le plasmide réplicatif pINA 250.

Les résultats présentés dans le tableau XI montrent que l'on peut obtenir avec l'ars18 une amplification corrigée d'un facteur 3,75 à 8,2.

TABLEAU XI

| SOUCHES | PLASMIDES | CARACTERISTIQUES | MILIEU | ACTIVITE INFα | % LEU+ | AMPLIFICATION CORRIGEE |
|---|---|---|---|---|---|---|
| JM77 | pINA187 | INTEGRATIF | YPDm | 100 | 100% | 1 |
| JM85 | pINA250 | REPLICATIF | YPDm | 300 | 49% | 6.1 |
| JM86 | pINA250 | REPLICATIF | YPDm | 300-225 | 36% | 8.2-6.2 |
| JM87 | pINA250 | REPLICATIF | YPDm | 225 | 60% | 3.75 |

Comparaison de la production d'activité antivirale sécrétée par la souche JM77 qui possède une copie chromosomique du gène hybride XPR2-INF 1 et les transformants JM85, JM86 et JM87 qui présentent le gène XPR2-INF 1 sur un plasmide réplicatif. La culture est réalisée à 20°C en milieu YPDm (extrait de levure 1 g/l protéose peptone 50 g/l, glucose 10 g/l). La culture est ensemencée à 10 unités Klett et les surnageants ont été prélevés 48 et 53 heures après l'ensemencement. L'activité antivirale est déterminée selon la méthode décrite par la Bonnardière et Laure (28) et est exprimée ici en unités arbitraires.

## EXEMPLE 14

## EXPRESSION ET AMPLIFICATION DU GENE CODANT POUR LA PROCHYMOSINE

La production de prochymosine par la levure Y. lipolytica a déjà été documentée dans le brevet cité en référence 23 et dans la publication de Franke et coll. (20), où le plasmide intégratif LEU2-proREN a été décrit. Ce plasmide, appelé pLX34, est représenté sur la Figure 14. Il possède le gène proREN sous le contrôle du promoteur du gène LEU2 de Y.lipolytica. La production de chymosine a été comparée dans :

- la souche DL 249, qui héberge pLX34 intégré au locus génomique bio ;
- la souche DL118, qui lui est isogénique, et qui a été transformée par le plasmide pINA184. Ce dernier dérive de pLX34 par adjonction de ars18 (figure 14). Des transformants produisant de la prochymosine et ayant le phénotype Leu$^+$ instable ont été sélectionnés et cultivés sur milieu liquide à 23°C.

Le tableau XII montre qu'après 60 heures de culture sur milieu minimum YNBS (yeast nitrogen base 6,7 g/l, glucose 10 g/l), la production de chymosine est plus élevée chez les transformants réplicatifs que chez le témoin intégratif par un facteur 1,3.

TABLEAU XII

| RECEPTRICE | TRANSFORMANTS | PLASMIDES | MODE | MILIEU | ACTIVITE CHYMOSINE | | % LEU+ | AMPLIFICATION CORRIGEE |
|---|---|---|---|---|---|---|---|---|
| DL118 | DI249 | pLX34 | Intégratif | YNBS | 1.0 | | 100% | |
| | TF1 | pINA184 | Réplicatif | - | 1.26 | | 70% | |
| | TF2 | - | - | - | 1.23 | Moyenne: 1.24 | - | |
| | TF4 | - | - | - | 1.29 | | - | 1.77 |
| | TF5 | - | - | - | 1.18 | | - | |
| INAG33122 | LX2-1 | pLX34 | Intégratif | ELPG | 1.0 | | 100% | |
| | 2-36 | pINA184 | Réplicatif | - | 1.28 | | 45% | 2.84 |

Dosage de l'activité chymosine presente dans des surnageants de culture de différents transformants de Y.lipolytica. L'activité est exprimée en unités arbitraires. Pour le dosage, on a suivi la méthode décrite dans la référence (20) : après centrifugation des cellules, le surnageant a été activé par un traitement de 20 minutes à pH 2 environ (avec acide chlorhydrique) afin de transformer le zymogène en chymosine active. L'activité de cette dernière à été mesurée par sa capacité à faire cailler 1 ml de lait (Difco skim milk à 12 % dans l'acétate de sodium 41, 5 mM, pH 6,5, chlorure de calcium 13,6 mM) à 37° C par comparaison avec une gamme standard de rennine commerciale (Sigma R 4879). Si l'on rapporte cette production aux cellules qui possèdent le plasmide sur milieu minimum (valeur moyenne de 70 %), on trouve un facteur d'amplification de 1,77.

On a réalisé la même expérience en transformant la souche INAG33122 (Fil$^+$), décrite précédemment par les mêmes plasmides pLX34 (intégration dirigée au locus génomique LEU2 par linéarisation du plasmide par l'enzyme XhoI) et pINA184 (plasmide réplicatif). Les transformants ont été cultivés sur milieu complet ELPG (extrait de levure 0,1 %, peptone 5%, glucose 1%). Après 50 heures de culture à 23°C la production de chymosine dans le milieu a été trouvée de nouveau 1,3 fois plus élevée dans le transformant réplicatif que dans le transformant intégratif. Après correction par le pourcentage de cellules $Leu^+$ observé à ce stade de la culture (45 %), on constate que l'amplification réalisée est d'un facteur 2,8.

Le gène INF-$alpha_1$ et les anticorps ont été communiqués par Claude La Bonnardière de l'Institut National de la Recherche Agronomique (INRA) de Jouy-en-Josas, de même que les dosages biologiques de l'activité de l'interféron ont été réalisés au sein de son laboratoire.

Les expériences complémentaires sur l'amplification de SUC2, INF-$alpha_1$ et la protéase ont été réalisées grâce au concours de Jean-Marc Nicaud.

Les souches suivantes ont été déposées, le 21 janvier 1988, à la Collection Nationale de Cultures de Microorganismes de l'Institut Pasteur, 28 rue du Docteur-Roux - 75724 Paris Cédex 15 :

- Escherichia coli : INAG 20 580/pINA123 sous le n° I-724
- Yarrowia lipolytica : INAG 33 129 sous le n° I-725
- Yarrowia lipolytica : INAG 33462/pINA119 sous le n° I-726.

## REFERENCES BIBLIOGRAPHIQUES


1. Williamson D.H.: Yeast, 1985, 1, (1-2), 1-14.

2. Kearsey S.: Bio Essays, 4, (4), 157-161.

3. Gaillardin C., Ribet A-M., Heslot H.: Curr. Genet., 1985, 10, 49-58.

4. Davidow L.S., Apostolakos D., O'Donnell M.M., Proctor A.R., Ogrydziak D.M., Wing R.A., Stasko I., De Zeeuw J.R.: Curr. Genet., 1985, 10, 39-48.

Gaillardin C., Ribet A-M.,: Curr. Genet., 1987, 11, 369-375.

6. Wing R.A., Ogrydziak D.M.: in Molecular Genetics of Filamentous Fungi, Timberlake W.E. (Ed.), UCLA Symposia vol. 34, 1985, 367-382.

7. Murray A.W., Szostak J..W.: Cell, 1983, 34, 961-970.

8. Davidow L.S., De Zeeuw J.R.,: European Patent Publication number 0 138 508-A1, 1984.

9. Fournier P., Gaillardin C., Persuy M-A., Klootwijk J., van Heerikhuizen H.: Gene, 1986, 42, 273-282.

10. Southern E.M.: J. Mol. Biol., 1975, 98, 503.

11. Chan C.S.M., Tye B-K.: J. Mol. Biol., 1983, 168, 505-523.

12. Miller J.H.: in Experiments in Molecular Genetics. Cold Spring Harbor Laboratory, Cold Spring Harbor, 1971.

13. Bradford H.M.,: Anal. Biochem., 1976, 72, 248.

14 Xuan J., Fournier P. et Gaillardin C. Cloning of the LYS5 gene encoding saccharopine dehydrogenase from the yeast Yarrowia lipolytica by target integration. Curr. Genet. 14 : 15-21 (1988).

15 Orr Weaver T. et Szostak J. Yeast recombination : the association between double-strand gap repair and crossing-over. Proc. Natl. Acad. Sci. 80 :4417-4421 (1983).

16 Kunes S. Botstein D. et Fox M. Transformation of yeast linearized DNA formation of inverted dimers and recombinant plasmid products. J. Mol. Biol. 184:375-387 (1985).

17 Davidow L., O'Donnell M., KaczmareK F., Pereira D., DeZeeuw J., Franke A. Cloning and sequencing of the alkaline extracellulaire protease gene of Y. lipolytica. J. Bacteriol. 169:4621-4629 (1987).

18 Taussig R. et Carlson M. Ncucleotide sequence for the yeast SUC2 gene for invertase. Nucleic Acids Res. 11 : 1943-1954 (1983).

19 Lefevre F. et la bonnardière C. Molecular cloning and sequencing of a gene encoding biologically active porcine interferon. J. of interferon Research 6:349-360 (1986).

20 Franke A., Kaczmarek F., Eisenhard M., Geoghegan K., Danley D., DeZeeuw J., O'Donnell M., Gollaher M. et Lance S. Expression and secretion of bovine prochymosin in Yarrowia lipolytica. Dev. in Industrial microbiology 29 : 43-57 (1988).

21 Ogrydziak D., Demain A. et Tannenbaum S. Regulation of extracellular protease production in Candida lipolytica. Biochim. Bioph. Acta 497:525-538 (1977).

22 Werner W., Rey H. et Wielinger H. Z. Analyt. Chem. 252 : 224 (1970).

23 Davidow L., Franke A., DeZeew J., Expression and secretion of heterologous proteins by Yarrowia lipolytica. transformants European patent application 86-307839,1986.

24 Kikuchi Y. Yeast plasmid requires a cis-acting locus and two plasmid proteins for its stable maintenance. Cell 35:487-493 (1983).

25 Wolf-Dietrich H. Sipiczki M. et Kohli J. Replicating plasmids in Schizosaccharomyces pombe : improvement of symmetric segregation by a new genetic element. Mol. Cell. Biol. 6:80-89 (1986).

26 Williamson D. The yeast ARS element, six years on : a progress report. Yeast 1 : 1-14 (1985).

27 Donnelly et al. $^{14}C$ methylated βcasein as a substrate for plasmin and its application to the study of milk protein transformations. B.B.A. 626:117-126 (1980).

28 La Bonnardière C. et Laude H. High interferon titer in newborn pig intestine during experimentally induced viral enteritis. Infect. Immun. 32:28-31 (1981).

induced viral enteritis. Infect. Immun. 32:28-31 (1981).

**Revendications**

1) Séquence ars efficace dans Yarrowia lipolytica.

2) Séquence ars selon la revendication 1, caractérisée en ce qu'elle est portée par le fragment d'ADN de 2,2 kb (BamHI/BamHI) présentant la carte de restriction de la figure 8B.

3) Séquence ars selon la revendication 1, caractérisée en ce qu'elle correspond à la partie efficace de la séquence décrite à la figure 13.

4) Séquence ars selon la revendication 1, caractérisée en ce qu'il s'agit d'une séquence ars68 ou ars77.

5) ADN comprenant une séquence ars selon l'une des revendications 1 à 4.

6) Plasmide comprenant une séquence ars selon l'une des revendications 1 à 4.

7) Plasmide portant une séquence ars efficace chez Yarrowia lipolytica selon l'une des revendications 1 à 4 et qui peut être obtenu notamment par le procédé suivant :

a) on constitue une banque de fragments génomiques de Y. lipolytica dans un vecteur intégratif complémentant une auxotrophie de Y. lipolytica ;

b) on transforme une souche hôte de Y. lipolytica, présentant une auxotrophie pouvant être complémentée par le vecteur intégratif précédent, par les plasmides de la banque précédente ;

c) on hybride sur colonie avec une sonde détectant le vecteur intégratif et on sélectionne les transformants présentant le signal le plus fort ;

d) on effectue un minilysat des clones vecteurs et on met en évidence les plasmides extrachromosomiques ;

e) ces plasmides portent une séquence ars que l'on peut confirmer en testant leur pouvoir de transformation qui est au moins 5 fois supérieur au pouvoir de transformation du vecteur d'origine.

8) Plasmide selon la revendication 7 pour lequel le procédé comprend les étapes suivantes :

- constitution d'une banque de fragments génomiques d'Y. lipolytica dans un vecteur intégratif, type pINA62 ;
- transformation de plasmides circulaires surenroulés de cette banque dans une souche Fil+, sélection des transformants LEU+ (peuvent contenir un plasmide intégré ou des plasmides réplicatifs) ;
- hybridation sur colonies avec une sonde pBR322 : on s'attend à trouver un signal plus fort dans les transformants réplicatifs multicopie ; isolement de clones ars potentiels ;
- minilysats des clones retenus, transfert de Southern d'ADN non restreint : mise en évidence de plasmides extrachromosomiques avec une sonde pBR322 ;
- transformation d'E. coli avec l'ADN des minilysats et amplification des plasmides ars ;
- caractérisation éventuelle de l'instabilité des clones.

9) Plasmide selon la revendication 8, caractérisé en ce que pour une souche hôte Fil- on teste, en outre, l'instabilité des transformants et on sélectionne les transformants présentant la plus faible stabilité.

10) Séquence d'ADN portant une séquence ars obtenue à partir d'un plasmide selon l'une des revendications 8 et 9.

11) Plasmide comportant un vecteur d'intégration complémentant une auxotrophie de Y. lipolytica comportant une séquence ars efficace dans Y. lipolytica.

12) Plasmide d'expression d'une protéine industrielle dans Y. lipolytica, caractérisé en ce qu'il comporte l'ensemble des éléments permettant d'assurer l'expression de ladite protéine dans Y. lipolytica, et en ce qu'il comporte une séquence ars.

13) Plasmide selon la revendication 12, caractérisé en ce qu'il comporte la séquence codant pour ladite protéine sous le contrôle d'une protéine de Y. lipolytica.

14) Plasmide selon la revendication 13, caractérisé en ce que le promoteur est le promoteur XPR2 ou de LEU2.

15) Plasmide selon la revendication 14, caractérisé en ce que la protéine est choisie parmi l'AEP, l'invertase, l'interféron-$\alpha$1 de porc, la prochymosine.

16) Plasmide selon la revendication 15, caractérisé en ce qu'il comporte le gène XPR2 de Y. lipolytica

17) Plasmide selon la revendication 15, caractérisé en ce qu'il comporte le gene Suc2 de S. cerevisiae sous le controle du promoteur XPR2.

18) Plasmide selon la revendication 17, caractérisé en ce que le gène Suc2 est fusionné à la séquence signal de la protéase alcaline extracellulaire.

19) Plasmide selon la revendication 15, caractérisé en ce que le gène proRENest sous le contrôle du promoteur du gène LEU2 de Y. lipolytica.

20) Souche de Y. lipolytica transformée par un plasmide selon l'une des revendications 6 à 9 et 11 à 19.

21) Souche selon la revendication 20, caractérisée en ce que ledit plasmide comporte au moins une séquence hétérologue.

22) Souche selon l'une des revendications 20 et 21, caractérisée en ce qu'il s'agit d'une souche Fil+.

23) Souche selon l'une des revendications 20 et 21, caractérisée en ce qu'il s'agit d'une souche Fil-.

24) Procédé de préparation d'une protéine industrielle, caractérisé en ce qu'on cultive, sur un milieu assurant sa croissance, une souche de Y. lipolytica selon l'une des revendications 20 à 23.

25) Protéine obtenue par la mise en oeuvre du procédé selon la revendication 24.

FORME MYCELIENNE
C
A
FORME LEVURE
C
A
C

FIG_1

A
B
PARENT
MUTANT

FIG_2

A
B

FIG_3

Bgl II
Bgl II
EcoRI
BamHI
Sal I
pINA 62
9.6 kb
EcoRI
ori
EcoRI
Sal I
EcoRI

FIG_4

A
B C D E F
G H I J K
O.C.
CHROMOSOME
C. C. C.

FIG_5

pINA 119
pINA 123
pINA 124
pINA 125
pINA 126
B/G
E
H
B
G


TAILLE EN KB

| pINA | PLASMIDE | INSERTION |
|---|---|---|
| 119 | 14.8 | 5.2 |
| 123 | 16.2 | 6.6 |
| 124 | 27.4 | 17.8 |
| 125 | 14.5 | 4.9 |
| 126 | 17.2 | 7.6 |

FIG_6

1kb
ARS
LEU 2
A
12.5
2.2
B
6.5
2.15
6.05
G
8.6
3.2
.3
2.6
E
3.7
5.4
2.8
1.45
95
.3
H
3.73
97
2.3
7.7
P
2.95
1.55
1.02
S
4.4
4.4
.7
5.2
X
5.7
3.2
1.8
2.6
1.3
SEQUENCES DELETEES
B B
H H H
SEQUENCES RECLONEES
B H
B B
H H
pINA 171
TRANSFORMATION


FIG_7

A
T7 PROMOTEUR
Sac I
Bat XI
Sac II
3'
5'
Eag I
Noc I
Xab I
Spc I
Bam HI
B
ARS
B
Sma I
CENTRAL
Pst I
Eco RI
Eco RV
5'
Hind III
Cla I
Sal I
Hind II
Acc I
Xho I
S
LEU2
Dra II
Apa I
A
Kpn I
3'
T3 PROMOTEUR
3.0 kb
M13 IG
"BLUESCRIPT M13 (phagemid)"
AmpR
lacZ
Col E1 ORI

B
B R N H T T B
C T V C R C
100bp

FIG_8

BamHI / Sau3A
Sau3A / BamHI
Pst I
BLA
LACZ
pINA 98
16 KB
Sal I
LEU2
Bgl II
Bgl II
ARS
Sal I

FIG_9

Transformation à haute fréquence et instabilité du caractère Leu+
Bgl II
Bgl II
Bgl II
Bgl II
pINA 123
oui
pINA 242
non
pINA 243
non
pINA 248
oui
Localisation probable de ars68
Bgl II
Bgl II
BamHI
BamHI
Bgl II
Bgl II
pINA 124
oui
pINA 294
non
pINA 241
non
Localisation probable de ars77
1kb
pBR322
insertion "ars"
LEU2

FIG_10

Hind III
EcoRV
BamHI
AsuII
Sau3A
AsuII
Sau 3A
BglII
BamHI
p INA 147
p INA 122
p INA 120
p INA 173
p INA 232
p INA 174
p INA 175
p INA 176
p INA 211
++ +
− −
− +
+ −
++ +
+ +
+ +
++ +
++ +
Transformation(?)
Réplication(?)
100 bp
Transformation a haute frequence (1)
instabilité du caractere Leu+(2)


FIG_11

EcoRI
Sal I
Hind III
10
2
p INA 232
11,3Kb
Bam HI
8
ars18
Hind III
Sal I
LEU2
4
EcoRI
6
EcoRI
Bgl II
BamHI
6
bla
5
1
Hind III
ars18
p INA 176
6,2 Kb
BamHI
2
Hind III
4
LEU2
Sal I
ApaI
3
Eco RI
SphI
EcoRI
7
1
tet
LEU2
pINA 240
8 Kb
6
2
EcoRI
bla
ars18
5
ori
3
4
SphI
EcoR!
SphI
EcoRI
7
1
LEU2
tet
6
pINA 240'
8 Kb
2
SphI
EcoRI
bla
ars18
5
ori
3
4
EcoRI
BamHI
SphI
EcoRI
7
1
LEU2
tet
pINA 237
8Kb
6
2
ars18
ori
bla
5
3
4
SphI
EcoRI
Eco RI
BamHI
SphI
EcoRI
7
1
LEU2
tet
6
pINA 237'
8Kb
2
SphI
ars18
ori
bla
5
3
4
EcoRI
Vecteur (pBR 322 ou Bluescript)
ars 18
LEU 2

FIG_12

```
BamHI
GGATCCCAAT ATTACACCCA AGTAGCATGC ATAAGCTAAA AGTAACTCGC AGCGCACACC   60

GTGCAGATTC ATAAGTCTAT GATTAATTGA ACGCCAATAA CCCGGCTTAC TACAAGTACA  120
                                                     J-10
AGTAGGTATA CATAGCGGTA ATGAATCATT AGAAAAAAAA AAAACAAAAA AAAACAAAAC  180
                  H-11     F-10
AAACTGTTGT GGATGCATCA ACAGTAGTAC ATAGTTGTAC GATGTACTTG TACTTGTAAA  240
                                             AsuII
AGCAAAAATG TACAATATCT CAGGGAGCGC AACTTTTACG TTCGAAGAAC AATGTACCGC  300

ATACCGCATT CTAGATTCTG CGGAACGTCT AACCTGGAAA TACGATTTTT TTTTTCTTTC  360

ATTTTTTTTG CTTCTTCAAA AGTATGGTAA TTTCCTACCA TTACAGTTGA CACTGAACGA  420
                            L-10
GGGGGGATTG AATTTAAGCA AAAAATTAAA TCAAAATACC TTTATGTATC CAGCCCATGT  480
                                                    Sau3A
AATAAACAAA AGGATTATAT AACAAGAAAT AAATATATAC CTTTAATGGA TCATTAGAAT  540
             D-11                             L-10         F-10
              K-10             HindIII
AAAAATAAAT ACGAGAAGCA CACCAGAGAA GCTTTTTGAT TGCCACTATA CCGCTACTTT  600
  E-11
GGTATATCTT ATTATAATTG TTGAATTTGC AAGATAGAAT GTCATTCATT GGAGAGAAAT  660

CCAAGGAATA TGTGGGATGA AATGACTAGA AGTATGAACA ATGAGAATAG TACATACTTG  720

TACCTGTATT TCTAGAAGAG AGAAAGACAG TTGAGTGTGT GATTCTCGTC CAATAATAAT  780
                                                         M-10
CTCAATAGTA ACGTGTGAAT AGCTGTTCTT TGATAGTTGA TATTTCTCGA TGACTATTTA  840
                                 AsuII                  EcoRV
TGTTGTACAA GGGATTTTTT TCGTTGCTGT TGATTTCGAA TTAGGCAATG CAGATATCAT  900

TTATGCTATC CATATTTAAG ATTTCCCATA CGCATTTATA ACATTTATTC TACATAAATT  960
                                                          G-10
GTTAAATGAA CGAACTGCCA TTATAAATTG TTTCCTAAAT AGGAAGTGTT TTTCATAAAG 1020
G-10   I-10                G-10
CAAGTAAGTT GTCTAATAAT ACTAAGTAAT AAAAATAAGT TCATACAATA TATTTTGAGA 1080
  I-10        M-10        H-11       E-11          K-10      A-12
                                                              J-10
ACATCATTTG GAGGCGGTAG ATGGAGTCTG TTTATTATTA AACAATGCGA GATGACCCCT 1140
   J-10      B-10         C-13
TAAATATTGA GAACATCAGT TGGAGGCGGC AGATGGAGTC TGTCTATTTA GCAATGGGAC 1200
          A-12          B-10           C-13
ATGACTGTCA GTATCATCAT CATGTATATA TATAATACAT ATAATATTAT ATAACACGAT 1260
                                           Sau3A        D-11
TTTTTTAAAT TATTGGCCCG AAAATTAATC AGTGTAGACT GGATC    1305
```

FIG_13

1-gene XPR2
a) construction de pINA 157
Eco RI
Cla I
Bgl II
Hind III
Xha I
Bgl II
Tet
XPR2
ori
Mlu I
Cla I
Pst I
pINA 157
7,1 Kb
EcoRI
BamHI /Sau 3A
Bgl II
bla
Sal I
LYS5
EcoRI
LEU2
Sph I
Bgl II
EcoRI
pINA 128
15Kb
pINA 157
13,1Kb
LYS5
Sph I
Mlu I
b) construction de pINA136
pINA 152
1,4 Kb
BamHI
Amp
ars18
pINA 171
11,8 Kb
ars 18
XPR 2
LEU2
LYS5
pINA 136
16,3Kb
2-gene SUC2
vecteurs pINA 169 et pINA 181
Apa I
pINA176
6,2 Kb
SUC2
pINA 169
12,7Kb
ars 18
XPR 2
LEU2
SUC2
pINA 181
9,9 Kb
FIG
14-1 et 14-2

3 - gene pro - INFα1

Hind III
Sal I
ori
URA3
Amp
Sal I
EcoRI
pINA 156
4,2 Kb

Eco RI
Pst I
Amp
ori
INFα
XPR2
Hind III
Cla I
MluI
Sal I
Apa I
Bgl II
Xba I
pINA 186
6,1 Kb

EcoRI
Cla I
BamHI
Sph I
Eco RI
Tet
LEU2
ars 18
ori
bla
SphI
EcoRI
Pst I
pINA 237
8Kb

a) construction de pINA 187

EcoRI
Pst I
Amp
URA3
ori
INFα
XPR2
Hind III
Cla I
MluI
Sal I
ApaI
XbaI
Bgl II
pINA 187
6,7 Kb

b) construction de pINA 250

EcoRI
Cla I
MluI
EcoRI
Bgl II
XPR2
LEU2
INFα1
Xba I
Apa I
ars 18
ori
bla
Sal I
SphI
EcoRI
PstI
pINA 250
8,6 Kb

ars 18
XPR2
LEU2
INF 1
URA3

4 - gene proREN
Cla I
Tet
proREN
ori
LEU2
Amp
Pvu I
plasmides pLX34 et pINA 184
pLX34
9,2 Kb
Cla I
BamH I
Hind III
Pvu I
Amp
ars 18
ori
HindIII
BamHI
pINA 130
6,5 Kb
BamHI
Cla I
HindIII
Bam HI
ars18
proREN
LEU2
ori
Amp
PvuI
pINA 184
11,4 Kb
ars18
LEU2
REN

FIG 14-3 et 14-4

Pst I
Cla I
Mlu I
Bgl II
Sal I
Sal I
Sal I
Bgl II
Eco RI
pINA 157
LYS 5
Pst I
Cla I
Mlu I
Bgl II
XbaI
Bgl II
Eco RI
Bgl II
Pst I
C
XPR 2

FIG_15

A
B
C
Croissance cellulaire (KLETT)
Activité protéasique (unité arbitraire)
TEMPS (H)
R
I
1000
500
100
50
10
5
10
20
30
40
50
FIG_16

Cla I
Hind III
Cla I
EcoRV
p INA 150
Insertion de l' Hind III - Eco RI fragment de XPR 2 dans pBR322
deletion Cla I
Xho II
BamHI
EcoRV
Sal I
Bgl II
phage M13 mp18-X4
phage avec mutation
Pst I
Hind III
Insertion du fragment BamHI-SalI de XPR2 avec mutation
Cla I
EcoRV
pINA 151
Cla I
EcoRI
BamHI
EcoRV
Hind III / Pst I
Sal I
pINA 165
Deletion EcoRV
Cla I
EcoRV
Hind III / Pst I
Sal I
pINA 166
Insertion du gene LEU 2
Cla I
Hind III
Sal I
p INA 167
Insertion de SUC2
Hind III
pINA169
Sal I
Hind III
Sal I
XPR 2 promoteur
LEU 2 gene
SUC 2 gene

FIG_17

A
1000
500
100
50
10
5
3
2
1
R
I
Croissance cellulaire (KLETT)
Activité invertase (unité arbitraire)
10
20
30
40
TEMPS (H)
B
1000
500
100
50
10
5
3
2
1
R
I
Croissance cellulaire (KLETT)
Activité invertase (unité arbitraire)
10
20
30
40
TEMPS (H)

FIG_18

FIG_19
EcoRI
Pst I
Amp
pLDG7
5,5Kb
ori
Hind III
Cla I
INFα
Sal I
BamHI
Xba I
Apa I
BamHI
Insertion d'un adaptateur
Xba I - Apa I
EcoRI
Pst I
Amp
pINA 185
5,5Kb
ori
INFα
Hind III
Cla I
Sal I
Bam HI
Xba I*
Apa I
Bam HI
1,9Kb Cla I - Xba I
EcoRI
Cla I
Hind III
Bgl II
Xba I
Bgl II
XPR2
Tet
pINA 154
7,1Kb
ori
Mlu I
Cla I
Pst I
EcoRI
Pst I
Amp
pINA 186
6,1Kb
ori
XPR2
INFα
Hind III
Cla I
Mlu I
Sal I
Bgl II
Apa I
Xba I
1,7Kb Eco RI -
Hind III
Hind III
Sal I
ori
URA3
pINA156
4,2Kb
Amp
Sal I
EcoRI
EcoRI
Pst I
Amp
URA3
pINA187
6,7Kb
ori
INFα
XPR2
Hind III
Cla I
Mlu I
Sal I
Apa I
Xba I
Bgl II
pBR 322
XPR 2
URA 3
pro INFα1

Schema de construction du plasmide pINA 187 portant la fusion pre-pro XPR2-INFα sous le controle du promoteur XPR2

Office européen des brevets

# RAPPORT DE RECHERCHE EUROPEENNE

Numero de la demande

EP 89 40 0218

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DISSERTATION ABSTRACTS INTERNATIONAL, vol. 49, no. 4, octobre 1988, page 1011-B, Ann Arbor, Mich., US; R.A. WING: "Development of the molecular biology of the yeast Yarrowia lipolytica: I. Development of a transformation system and search for autonomously replicating sequences. II. Cloning and sequencing of the alkaline extracellular protease structural gene" * University of California, Davis, 1987 * | 1,5,6,7,20,25 | C 12 N 15/00<br>C 12 N 1/16<br>C 12 P 21/02 |
| A | EP-A-0 045 573 (THE BOARD OF TRUSTEES OF LELAND STANFORD JUNIOR UNIVERSITY) | | |
| A | EP-A-0 180 899 (PHILIPS PETROLEUM CO.) | | |
| D,A | EP-A-0 220 864 (PHIZER INC.) | | |

DOMAINES TECHNIQUES RECHERCHES (Int. Cl.4)

C 12 N
C 12 P

Le présent rapport a été établi pour toutes les revendications

| Lieu de la recherche | Date d'achèvement de la recherche | Examinateur |
|---|---|---|
| LA HAYE | 18-05-1989 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)